# EUROPEAN PATENT APPLICATION

(11) **EP 3 072 940 A1**
(43) Date of publication of application: **28.09.2016**
(21) Application number: 15177406.4
(22) Date of filing: 17.07.2015
(51) Int. Cl.: C09K 11/02, C09K 11/56, C09K 11/88, G01N 33/58

(54) **CONTINUOUSLY EMISSIVE CORE/SHELL NANOPLATELETS**

(30) Priority: 27.03.2015 US 201562139152 P
(71) Applicant: Nexdot, 75005 Paris (FR)
(72) Inventor: Heuclin, Hadrien, 75005 Paris (FR); Nadal, Brice, 75005 Paris (FR); Grazon, Chloé, 75005 Paris (FR)
(74) Representative: Icosa

(57) **Abstract**

The present invention relates to a core/shell nanoplatelet and its use as a fluorophore or a fluorescent agent.

## Description

### FIELD OF INVENTION

The present invention relates to the field of nanoparticles and especially semiconductor nanocrystals. In particular, the present invention relates to core/shell nanoplatelets that exhibit desirable characteristics for use as imaging agents, especially bio-imaging agents, such as suppressed blinking.

### BACKGROUND OF INVENTION

Fluorescent dyes or fluorophores have a wide variety of uses including the labeling of proteins (e.g., antibodies), DNA, carbohydrates, and cells. Fluorescent-labeled substrates have been used for visualization and/or quantitative measurements in various applications including biology, medicine, electronics, biomedicine, and forensics. In particular, the investigation of fundamental process in life sciences relies on the fast, sensitive, reliable and reproductive detection of interplay of biomolecules with one another.

Semiconductor nanoparticles, such as quantum dots, are known as desirable fluorophores due to their interesting properties: high quantum yield, narrow fluorescence full width at half maximum, resistance to photobleaching, large absorption cross-section, tunable emission wavelength, etc. However, quantum dots exhibit fluorescence intermittency or blinking. Blinking severely limits the application of quantum dots as imaging agents, especially for real-time monitoring of fluorophore-labeled biomolecules. Therefore there is still a need for semiconductor nanoparticles exhibiting suppressed blinking.

### SUMMARY

The present invention thus relates to a population of fluorescent colloidal nanoplatelets, each member of the population comprising a nanoplatelet core including a first semiconductor material and a shell including a second semiconductor material on the surface of the nanoplatelet core, wherein at least 40% of the nanoplatelets of the population are continuously emissive for a period of at least one minute.

According to one embodiment, the shell of the nanoplatelet has a thickness of at least 3 nm.

According to one embodiment, after ligand exchange reaction the population exhibits a quantum yield decrease of less than 50%. According to one embodiment, the ligand is selected from a carboxylic acid, a thiol, an amine, a phosphine, a phosphine oxide an amide, an ester, a pyridine, an imidazole and/or an alcohol.

According to one embodiment, the population exhibits fluorescence quantum efficiency decrease of less than 50% after one hour under light illumination.

According to one embodiment, the population exhibits fluorescence quantum efficiency at 100 °C or above that is at least 80% of the fluorescence quantum efficiency of the population at 20°C. According to one embodiment, the temperature is in a range from 100 °C to 250°C.

According to one embodiment, the material composing the core and the shell comprises a material MxEy wherein:
M is selected from group Ib, IIa, IIb, IIIa, IIIb, IVa, IVb, Va, Vb, VIb, VIIb, VIII or mixtures thereof;
E is selected from group Va, VIa, VIIa or mixtures thereof; and
x and y are independently a decimal number from 0 to 5.

According to one embodiment, the material composing the core and the shell comprises a material MxEy, wherein:
M is Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is O, S, Se, Te, N, P, As, F, Cl, Br, I, or a mixture thereof, and
x and y are independently a decimal number from 0 to 5.

The present invention also relates to the use of a fluorescent colloidal nanoplatelet according to the invention.

The present invention also relates to a fluorophore conjugate comprising at least one fluorescent colloidal nanoplatelet according to the invention associated to a specific-binding component.

According to one embodiment, said specific-binding component is selected among antigens, steroids, vitamins, drugs, haptens, metabolites, toxins, environmental pollutants, amino acids, peptides, proteins, antibodies, polysaccharides, nucleotides, nucleosides, nucleic acids, nucleic acid polymers, carbohydrates, lipids, phospholipids, polymers, lipophilic polymers, polymeric microparticles, cells and virus.

The present invention also relates to the use of the fluorescent colloidal nanoplatelet or the fluorophore conjugate according to the invention in a detection system.

The present invention also relates to a method for detecting an analyte in a sample, said method comprising:
- contacting said sample with a fluorophore conjugate according to the invention, wherein the specific-binding component is a binding partner for said analyte;
- incubating said conjugate with said sample for a sufficient amount of time for said analyte and said component to interact, thereby forming a fluorescent analyte; and
- illuminating said fluorescent analyte with an appropriate wavelength, whereby the presence of said analyte is determined in said sample.

The present invention also relates to a method for detecting multiple analytes in a sample, said method comprising:
- contacting said sample with multiple fluorophore conjugates according to the invention, wherein each specific-binding component is a binding partner for one analyte and wherein each fluorophore presents a different fluorescence emission;
- incubating said conjugates with said sample for a sufficient amount of time for said analytes and said components to interact, thereby forming multiple fluorescent analytes;
- illuminating said fluorescent analytes with an appropriate wavelength, whereby the presence of said analytes is determined in said sample.

The present invention also relates to a kit for detection of at least one analyte in a sample comprising at least one fluorescent colloidal nanoplatelet or at least one fluorophore conjugate according to the invention.

The present invention also relates to the use of the fluorescent colloidal nanoplatelet or the fluorophore conjugate according to the invention for in vivo animal imaging or for ex vivo live cells imaging, in fluorescence detection methods or as biosensors.

### DEFINITIONS

In the present invention, the following terms have the following meanings:
- As used herein the singular forms **"a", "an",** and **"the"** include plural reference unless the context clearly dictates otherwise.
- The term **"about"** is used herein to mean approximately, roughly, around, or in the region of. When the term "about" is used in conjunction with a numerical range, it modifies that range by extending the boundaries above and below the numerical values set forth. In general, the term "about" is used herein to modify a numerical value above and below the stated value by a variance of 20 percent.
- **"Antibody"** is used herein includes antibodies obtained from both polyclonal and monoclonal preparations, as well as, the following: hybrid (chimeric) antibody molecules; F (ab') 2 and F (ab) fragments; Fv molecules; single-chain Fv molecules (sFv); dimeric and trimeric antibody fragment constructs; minibodies; and, any functional fragments obtained from such molecules, wherein such fragments retain specific binding properties of the parent antibody molecule.

- **"Aptamer"** (or nucleic acid antibody) refers to a single-or double-stranded DNA or a single-stranded RNA molecule that recognizes and binds to a desired target molecule by virtue of its shape.
- "**Barcode**" refers to one or more sizes, size distributions, compositions, or any combination thereof, of the fluorophores of the invention. Each size, size distribution and/or composition of the fluorophores of the invention has a characteristic emission spectrum, e. g., wavelength, intensity, FWHM, and/or fluorescent lifetime. In addition to the ability to tune the emission energy by controlling the size of the particular fluorophore of the invention, the intensities of that particular emission observed at a specific wavelength are also capable of being varied, thus increasing the potential information density provided by the fluorophore barcode system. In preferred embodiments, 2-15 different intensities may be achieved for a particular emission at a desired wavelength, however, one of ordinary skill in the art will realize that more than fifteen different intensities may be achieved, depending upon the particular application of interest. For the purposes of the present invention, different intensities may be achieved by varying the concentrations of the particular size of the fluorophore of the invention attached to, embedded within or associated with an item, compound or matter of interest. The "barcode" enables the determination of the location or identity of a particular item, compound or matter of interest. For example, the fluorophores of the invention can be used to barcode chromosomes, as well as portions of chromosomes, for spectral karyotyping, as described further below.
- **"Biological sample"** refers to a sample of isolated cells, tissue or fluid, including but not limited to, for example, plasma, serum, spinal fluid, semen, lymph fluid, the external sections of the skin, respiratory, intestinal, and genitourinary tracts, tears, saliva, milk, blood cells, tumors, organs, and also samples of in vitro cell culture constituents (including but not limited to conditioned medium resulting from the growth of cells in cell culture medium, putatively virally infected cells, recombinant cells, and cell components).

- **"Biomolecule"** refers to a synthetic or naturally occurring molecule, such as a protein, amino acid, nucleic acid, nucleotide, carbohydrate, sugar, lipid and the like.
- **"Blinking"** or **"Fluorescence intermittency"** refers to the interruption of fluorescence emission during one or more periods. Indeed continuously illuminated quantum dots emit detectable luminescence for limited times, interrupted by dark periods during which no emission occurs.
- **"Continuously emissive nanoplatelets"** over a predetermined period refers to nanoplatelets which exhibit, under excitation, fluorescence (or photoluminescence) intensity above a threshold over the predetermined period. The integration time is set to allow sufficient excitation events of the nanoplatelets and is superior or equal to 1 ms. According to the present invention, during a measurement (see examples), said threshold may be set at three times the noise.
- **"Fluorescence lifetime"** refers to the average time wherein an excited fluorophore remains in the excited state before it emits a photon and decays to the ground state.
- **"Fluorescence quantum efficiency or quantum yield"** refers to the ratio between the numbers of photons emitted by fluorescence divided by the number of absorbed photons.
- **"Homogeneous assay"** is one that is performed without transfer, separation or washing steps. Thus, for example, a homogeneous High Throughput Screening (HTS) assay involves the addition of reagents to a vessel, e. g., a test tube or sample well, followed by the detection of the results from that particular well. A homogeneous HTS assay can be performed in the solution in the test tube or well, on the surface of the test tube or well, on beads or cells which are placed into the test tube or the well, or the like. The detection system typically used is a fluorescence, chemiluminescence, or scintillation detection system.
- **"Imaging agent"** or **"fluorescent label", "fluorophore"** or **"dye"** are used interchangeably and refers to a fluorescent chemical compound that can re-emit light upon light excitation.
- The nanoplatelet fluorophore of the invention is **"linked"** or **"conjugated"** to, or **"associated"** with, a specific-binding molecule or member of a binding pair when the fluorophore is chemically coupled to, or associated with the specific binding molecule. Thus, these terms intend that the fluorophore of the invention may either be directly linked to the specific-binding molecule or may be linked via a linker moiety, such as via a chemical linker described below. The terms indicate items that are physically linked by, for example, covalent chemical bonds; physical forces such as van der Waals or hydrophobic interactions, encapsulation, embedding, or the like. As an example without limiting the scope of the invention, the fluorophore of the invention can be conjugated to molecules that can interact physically with biological compounds such as cells, proteins, nucleic acids, subcellular organelles and other subcellular components. For example, the fluorophore of the invention can be associated with biotin which can bind to the proteins, avidin and streptavidin. Also, the fluorophore of the invention can be associated with molecules that bind nonspecifically or sequence-specifically to nucleic acids (DNA, RNA). As examples without limiting the scope of the invention, such molecules include small molecules that bind to the minor groove of DNA, small molecules that form adducts with DNA and RNA; cisplatin, molecules that intercalate between the base pairs of DNA (e. g. methidium, propidium, ethidium, porphyrins, etc., radiomimetic DNA damaging agents such as bleomycin, neocarzinostatin and other enediynes and metal complexes that bind and/or damage nucleic acids through oxidation; chemical and photochemical probes of DNA.
- **"Monolayer"** refers to a film or a continuous layer being of one atom thick.
- **"Multiplexing"** refers to the implementation of an assay or other analytical method in which multiple analytes or biological states can be detected simultaneously by using more than one detectable label, each of which emits at a distinct wavelength, with a distinct intensity, with a distinct FWHM, with a distinct fluorescence lifetime, or any combination thereof. Preferably multiplexing is performed by making use of fluorophore-specific decay behavior (i.e. mono-exponential decay behavior), measured at a single excitation and a single emission wavelength, to discriminate between fluorophores. This latter approach requires sufficiently different lifetimes and preferably mono-exponential fluorescence decays. Preferably, each detectable label is linked to one of a plurality of first members of binding pairs each of which first members is capable of binding to a distinct corresponding second member of the binding pair. A multiplexed method using the fluorophores of the invention having distinct emission spectra can be used to detect simultaneously in the range of 2 to 1,000,000, preferably in the range of 2 to 10,000, more preferably in the range of 2 to 100, or any integer between these ranges, and even more preferably in the range of up to 10 to 20, e. g., 2, 3, 4, 5, 6, 7, 8, 9, 10, 11, 12, 13, 14, 15, 16, 17, 18, 19, or 20, of analytes, biological compounds or biological states.
- **"Nanoparticle or nanocrystal"** refers to a particle of any shape having at least one dimension in the 0.1 to 100 nanometers range.
- **"Nanoplatelet", "nanosheet", "nanoplate"** or **"2D-nanoparticle"** refers interchangeably to a nanoparticle having one dimension smaller than the two others; said dimension ranging from 0.1 to 100 nanometers. In the sense of the present invention, the smallest dimension (hereafter referred to as the thickness) is smaller than the other two dimensions (hereafter referred to as the length and the width) by a factor of at least 1.5, 2, 2.5, 3, 3.5, 4.5 or 5.
- **"On-time fraction":** refers to the fraction of time during continuous excitation in which a particle is exhibiting fluorescence emission, referred to as being "on".
- **"Polynucleotide", "oligonucleotide", "nucleic acid"** and **"nucleic acid molecule"** refers to a polymeric form of nucleotides of any length, either ribonucleotides or deoxyribonucleotides. This term refers only to the primary structure of the molecule. Thus, the term includes triple-, double- and single stranded DNA, as well as triple-, double- and single-stranded RNA. It also includes modifications, such as by methylation and/or by capping, and unmodified forms of the polynucleotide. More particularly, the terms "polynucleotide", "oligonucleotide", "nucleic acid" and "nucleic acid molecule" include polydeoxyribonucleotides (containing 2-deoxy-D-ribose), polyribonucleotides (containing D-ribose), any other type of polynucleotide which is an N-or C-glycoside of a purine or pyrimidine base, and other polymers containing nonnucleotidic backbones, for example, polyamide (e. g., peptide nucleic acids (PNAs)) and polymorpholino (polymers, and other synthetic sequence-specific nucleic acid polymers providing that the polymers contain nucleobases in a configuration which allows for base pairing and base stacking, such as is found in DNA and RNA). There is no intended distinction in length between the terms "polynucleotide", "oligonucleotide", "nucleic acid" and "nucleic acid molecule", and these terms will be used interchangeably. These terms refer only to the primary structure of the molecule. Thus, these terms include, for example, 3'deoxy-2', 5'-DNA, oligodeoxyribonucleotide N3'P5'phosphoramidates, 2'-O-alkyl- substituted RNA, double-and single-stranded DNA, as well as double- and single-stranded RNA, DNA: RNA hybrids, and hybrids between PNAs and DNA or RNA, and also include known types of modifications, for example, labels which are known in the art, methylation, "caps", substitution of one or more of the naturally occurring nucleotides with an analog, internucleotide modifications such as, for example, those with uncharged linkages (e. g., methyl phosphonates, phosphotriesters, phosphoramidates, carbamates, etc.), with negatively charged linkages (e. g., phosphorothioates, phosphorodithioates, etc.), and with positively charged linkages (e. g., aminoalklyphosphoramidates, aminoalkylphosphotriesters), those containing pendant moieties, such as, for example, proteins (including nucleases, toxins, antibodies, signal peptides, poly-L-lysine, etc.), those with intercalators (e. g., acridine, psoralen, etc.), those containing chelators (e. g., metals, radioactive metals, boron, oxidative metals, etc.), those containing alkylators, those with modified linkages (e. g., alpha anomeric nucleic acids, etc.), as well as unmodified forms of the polynucleotide or oligonucleotide. In particular, DNA is deoxyribonucleic acid.
- **"Polypeptide"** and **"protein"** refer to a molecular chain of amino acids linked through peptide bonds. The terms do not refer to a specific length of the product. Thus, "peptides", "oligopeptides" and "proteins" are included within the definition of polypeptide. The terms include post-translational modifications of the polypeptide, for example, glycosylations, acetylations, phosphorylations and the like. In addition, protein fragments, analogs, mutated or variant proteins, fusion proteins and the like are included within the meaning of polypeptide.
- **"Shell"** refers to a film or a layer of at least one atom thick covering the initial nanoplatelet on each faces (i.e. on the entire surface except, if the growth process is performed on a substrate, on the surface in contact with said substrate).
- **"Specific-binding molecule"**and **"affinity molecule"**are used interchangeably herein and refer to a molecule that will selectively bind, through chemical or physical means to a detectable substance present in a sample. By "selectively bind" is meant that the molecule binds preferentially to the target of interest or binds with greater affinity to the target than to other molecules. For example, an antibody will selectively bind to the antigen against which it was raised; a DNA molecule will bind to a substantially complementary sequence and not to unrelated sequences. The affinity molecule can comprise any molecule, or portion of any molecule, that is capable of being linked to a fluorophore of the invention and that, when so linked, is capable of recognizing specifically a detectable substance. Such affinity molecules include, by way of example, such classes of substances as antibodies, as defined below, monomeric or polymeric nucleic acids, aptamers, proteins, polysaccharides, sugars, and the like.

### DETAILED DESCRIPTION

This invention relates to a nanoplatelet comprising an initial nanoplatelet core and a shell.

According to a first embodiment, the initial nanoplatelet is an inorganic, colloidal, semiconductor and/or crystalline nanoplatelet.

According to one embodiment, the initial nanoplatelet has a thickness ranging from 0.3 nm to less than 500 nm, from 5 nm to less than 250 nm, from 0.3 nm to less than 100 nm, from 0.3 nm to less than 50 nm, from 0.3 nm to less than 25 nm, from 0.3 nm to less than 20 nm, from 0.3 nm to less than 15 nm, from 0.3 nm to less than 10 nm, or from 0.3 nm to less than 5 nm.

According to one embodiment, at least one of the lateral dimensions of the initial nanoplatelet is ranging from 2 nm to 1m, from 2 nm to 100 mm, from 2 nm to 10 mm, from 2 nm to 1 mm, from 2 nm to 100 µm, from 2 nm to 10 µm, from 2 nm to 1 µm, from 2 nm to 100 nm, or from 2 nm to 10 nm.

According to one embodiment, the material composing the initial nanoplatelet comprises a material MxEy, wherein:
M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is selected from O, S, Se, Te, N, P, As, F, Cl, Br, I, or a mixture thereof; and
x and y are independently a decimal number from 0 to 5.

According to an embodiment, the material MxEy comprises cationic element M and anionic element E in stoichiometric ratio, said stoichiometric ratio being characterized by values of x and y corresponding to absolute values of mean oxidation number of elements E and M respectively.

According to one embodiment, the faces substantially normal to the axis of the smallest dimension of the initial nanoplatelet consist either of M or E.

According to one embodiment, the smallest dimension of the initial nanoplatelet comprises an alternate of atomic layers of M and E.

According to one embodiment, the number of atomic layers of M in the initial nanoplatelet is equal to one plus the number of atomic layer of E.

According to an embodiment, the material composing the initial nanoplatelet comprises a material MxNyEz, wherein:
- M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- N is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- E is selected from O, S, Se, Te, N, P, As, F, Cl, Br, I or a mixture thereof; and
x, y and z are independently a decimal number from 0 to 5, at the condition that when x is 0, y and z are not 0, when y is 0, x and z are not 0 and when z is 0, x and y are not 0.

According to a preferred embodiment, the material composing the initial nanoplatelet comprises a material MxEy wherein:
M is selected from group Ib, IIa, IIb, IIIa, IIIb, IVa, IVb, Va, Vb, VIb, VIIb, VIII or mixtures thereof;
E is selected from group Va, VIa, VIIa or mixtures thereof; and
x and y are independently a decimal number from 0 to 5.

According to one embodiment, the material composing the initial nanoplatelet comprises a semi-conductor from group IIb-VIa, group IVa-VIa, group Ib-IIIa-VIa, group IIb-IVa-Va, group Ib-VIa, group VIII-VIa, group IIb-Va, group IIIa-VIa, group IVb-VIa, group IIa-VIa, group IIIa-Va, group IIIa-VIa, group VIb-VIa, or group Va-VIa.

According to one embodiment, the material composing the initial nanoplatelet comprises at least one semiconductor chosen among CdS, CdSe, CdTe, CdO, Cd₃P₂, Cd₃As₂, ZnS, ZnSe, ZnO, ZnTe, Zn₃P₂, Zn3As2, HgS, HgSe, HgTe, HgO, GeS, GeSe, GeTe, SnS, SnS₂, SnSe₂, SnSe, SnTe, PbS, PbSe, PbTe, GeS₂, GeSe₂, CuInS₂, CuInSe₂, CuS, Cu₂S, Ag₂S, Ag₂Se, Ag₂Te AgInS₂, AgInSe₂, FeS, FeS₂, FeO, Fe₂O₃, Fe₃O₄, Al₂O₃, TiO₂, MgO, MgS, MgSe, MgTe, AlN, AlP, AlAs, AlSb, GaN, GaP, GaAs, GaSb, InN, InP, InAs, InSb, In₂S₃,TlN, TlP, TlAs, TISb, Bi₂S₃, Bi₂Se₃, Bi₂Te₃, MoS₂, WS₂, VO₂ or a mixture thereof.

According to a preferred embodiment, the initial nanoplatelet is selected from the group consisting of CdS, CdSe, CdSSe, CdTe, ZnS, ZnSe, ZnTe, PbS, PbSe, PbTe, CuInS₂, CuInSe₂, AgInS₂, AgInSe₂, CuS, Cu₂S,Ag₂S, Ag₂Se, Ag₂Te, FeS, FeS₂, PdS, Pd₄S, WS₂ or a mixture thereof.

According to one embodiment, the initial nanoplatelet comprises an alloy of the aforementioned materials.

According to one embodiment, the initial nanoplatelet comprises an additional element in minor quantities. The term "minor quantities" refers herein to quantities ranging from 0.0001 % to 10% molar, preferably from 0.001 % to 10% molar.

According to one embodiment, the initial nanoplatelet comprises a transition metal or a lanthanide in minor quantities. The term "minor quantities" refers herein to quantities ranging from 0.0001 % to 10% molar, preferably from 0.001 % to 10% molar.

According to one embodiment, the initial nanoplatelet comprises in minor quantities an element inducing an excess or a defect of electrons compared to the sole nanoplatelet. The term "minor quantities" refers herein to quantities ranging from 0.0001% to 10% molar, preferably from 0.001% to 10% molar.

According to one embodiment, the initial nanoplatelet comprises in minor quantities an element inducing a modification of the optical properties compared to the sole nanoplatelet. The term "minor quantities" refers herein to quantities ranging from 0.0001 % to 10% molar, preferably from 0.001 % to 10% molar.

According to one embodiment, the initial nanoplatelet consists of a core/shell nanoplatelet such as a core/shell nanoplatelet known by one skilled in the art or a core/shell nanoplatelet according to the present invention. According to one embodiment, the "core" nanoplatelets can have an overcoating or shell on the surface of its core.

According to a first embodiment, the final nanoplatelet is an inorganic, colloidal, semiconductor and/or crystalline nanoplatelet.

According to one embodiment, the final nanoplatelet has a thickness ranging from 0.5 nm to 10 mm, from 0.5 nm to 1 mm, from 0.5 nm to 100 µm, from 0.5 nm to 10 µm, from 0.5 nm to 1 µm, from 0.5 nm to 500 nm, from 0.5 nm to 250 nm, from 0.5 nm to 100 nm, from 0.5 nm to 50 nm, from 0.5 nm to 25 nm, from 0.5 nm to 20 nm, from 0.5 nm to 15 nm, from 0.5 nm to 10 nm or from 0.5 nm to 5 nm.

According to one embodiment, at least one of the lateral dimensions of the final nanoplatelet is ranging from 2 nm to 1m, from 2 nm to 100 mm, from 2 nm to 10 mm, from 2 nm to 1 mm, from 2 nm to 100 µm, from 2 nm to 10 µm, from 2 nm to 1 µm, from 2 nm to 100 nm, or from 2 nm to 10 nm.

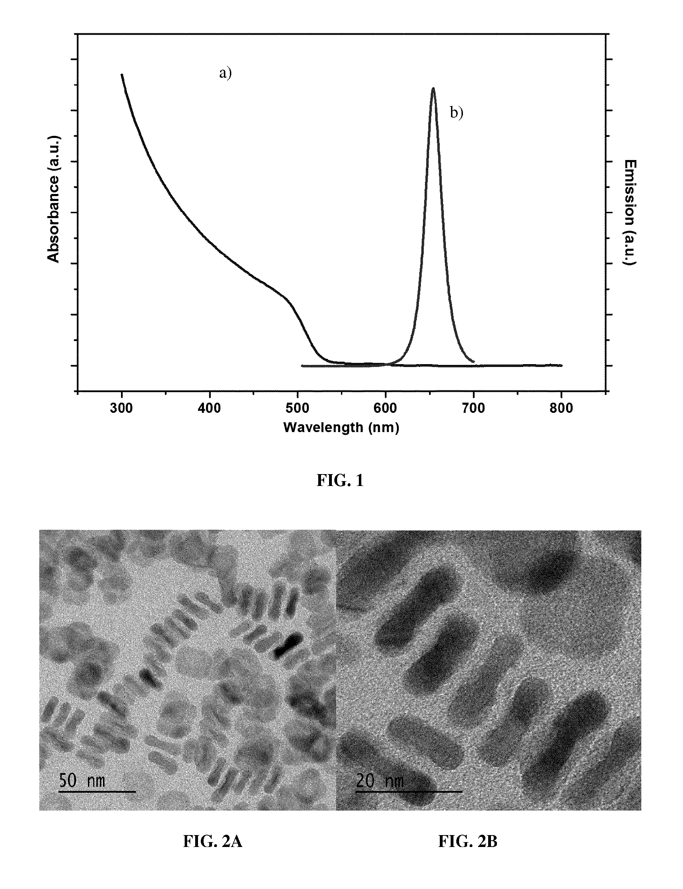

According to one embodiment, the thickness of the shell is ranging from 0.2 nm to 10 mm, from 0.2 nm to 1 mm, from 0.2 nm to 100 µm, from 0.2 nm to 10 µm, from 0.2 nm to 1 µm, from 0.2 nm to 500 nm, from 0.2 nm to 250 nm, from 0.2 nm to 100 nm, from 0.2 nm to 50 nm, from 0.2 nm to 25 nm, from 0.2 nm to 20 nm, from 0.2 nm to 15 nm, from 0.2 nm to 10 nm or from 0.2 nm to 5 nm **(****Figures 2****;** **3****;** **4****;** **11****;** **13****;** **14** and **16****).**

According to one embodiment, the material composing the shell comprises a material MxEy, wherein:
M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is selected from O, S, Se, Te, N, P, As, F, Cl, Br, I, or a mixture thereof,
and x and y are independently a decimal number from 0 to 5.

According to an embodiment, the material MxEy comprises cationic element M and anionic element E in stoichiometric ratio, said stoichiometric ratio being characterized by values of x and y corresponding to absolute values of mean oxidation number of elements E and M respectively.

According to one embodiment, the faces substantially normal to the axis of the smallest dimension of the shell consist either of M or E.

According to one embodiment, the smallest dimension of the shell comprises either an alternate of atomic layers of M and E.

According to one embodiment, the number of atomic layers of M in the shell is equal to one plus the number of atomic layer of E.

According to an embodiment, the material composing the shell comprises a material MxNyEz, wherein:
- M is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- N is selected from Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb or a mixture thereof;
- E is selected from O, S, Se, Te, N, P, As, F, Cl, Br, I, or a mixture thereof; and
x, y and z are independently a decimal number from 0 to 5, at the condition that when x is 0, y and z are not 0, when y is 0, x and z are not 0 and when z is 0, x and y are not 0.

According to a preferred embodiment, the material composing the shell comprises a material MxEy wherein:
M is selected from group Ib, IIa, IIb, IIIa, IIIb, IVa, IVb, Vb, VIb, VIIb, VIII or mixtures thereof;
E is selected from group Va, VIa, VIIa or mixtures thereof; and
x and y are independently a decimal number from 0 to 5.

According to one embodiment, the material composing the shell comprises a semi-conductor from group IIb-VIa, group IVa-VIa, group Ib-IIIa-VIa, group IIb-IVa-Va, group Ib-VIa, group VIII-VIa, group IIb-Va, group IIIa-VIa, group IVb-VIa, group IIa-VIa, group IIIa-Va, group IIIa-VIa, group VIb-VIa, or group Va-VIa.

According to one embodiment, the material composing the shell comprises at least one semiconductor chosen among CdS, CdSe, CdTe, CdO, Cd₃P₂, Cd₃As₂, ZnS, ZnSe, ZnO, ZnTe, Zn₃P₂, Zn3As2, HgS, HgSe, HgTe, HgO, GeS, GeSe, GeTe, SnS, SnS₂, SnSe₂, SnSe, SnTe, PbS, PbSe, PbTe, GeS₂, GeSe₂, CuInS₂, CuInSe₂, CuS, Cu₂S, Ag₂S, Ag₂Se, Ag₂Te AgInS₂, AgInSe₂, FeS, FeS₂, FeO, Fe₂O₃, Fe₃O₄, Al₂O₃, TiO₂, MgO, MgS, MgSe, MgTe, AlN, AlP, AlAs, AlSb, GaN, GaP, GaAs, GaSb, InN, InP, InAs, InSb, In₂S₃,TlN, TlP, TlAs, TISb, Bi₂S₃, Bi₂Se₃, Bi₂Te₃, MoS₂, WS₂, VO₂ or a mixture thereof.

According to one embodiment, the shell comprises an alloy of the aforementioned materials.

According to a preferred embodiment, the shell is selected from the group consisting of CdS, CdSe, CdSSe, CdTe, ZnS, ZnSe, ZnTe, PbS, PbSe, PbTe, CuInS₂, CuInSe₂, AgInS₂, AgInSe₂, CuS, Cu₂S, Ag₂S, Ag₂Se, Ag₂Te, FeS, FeS₂, PdS, Pd₄S, WS₂, or a mixture thereof.

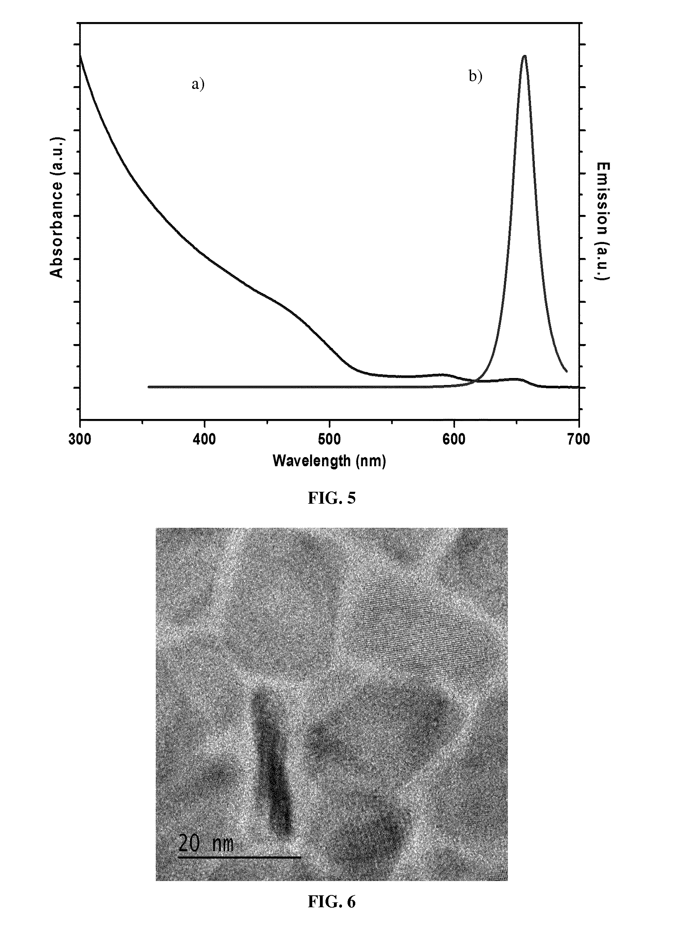

According to a preferred embodiment, the final core/shell nanoplatelet is selected from the group consisting of CdSe/CdS **(****Figure 1****);** CdSe/CdZnS **(****Figures 5; 6****;** **7** and **8****);** CdSe/ZnS; CdSeTe/CdS; CdSeTe/CdZnS; CdSeTe/ZnS; CdSSe/CdS; CdSSe/CdZnS; CdSSe/ZnS.

According to a preferred embodiment, the final core/shell nanoplatelet is selected from the group consisting of CdSe/CdS/ZnS; CdSe/CdZnS/ZnS; CdSeTe/CdS/ZnS; CdSeTe/CdZnS/ZnS; CdSeTe/ZnS; CdSSe/CdS/ZnS; CdSSe/CdZnS/ZnS; CdSSe/ZnS.

According to one embodiment, the final nanoplatelet is homostructured, i.e. the initial nanoplatelet and the shell are composed of the same material.

In one embodiment, the final nanoplatelet is heterostructured, i.e. the initial nanoplatelet and the shell are composed of at least two different materials.

According to one embodiment, the final nanoplatelet comprises the initial nanoplatelet and a sheet comprising at least one layer covering all of the initial nanoplatelet. Said layer being composed of the same material as the initial nanoplatelet or a different material than the initial nanoplatelet.

According to one embodiment, the final nanoplatelet comprises the initial nanoplatelet and a shell comprising 1, 2, 3, 4, 5, 6, 7, 8, 9, 10, 15, 20, 25, 50 or more monolayers covering all of the initial nanoplatelet. Said layers being of same composition as the initial nanoplatelet or being of different composition than the initial nanoplatelet or being of different composition one to another.

According to one embodiment, the final nanoplatelet comprises the initial nanoplatelet and a shell comprising at least 5, 6, 7, 8, 9, 10, 15, 20, 25, 50 or more monolayers covering all of the initial nanoplatelet. Said layers being of same composition as the initial nanoplatelet or being of different composition than the initial nanoplatelet or being of different composition one to another.

According to one embodiment, the faces substantially normal to the axis of the smallest dimension of the final nanoplatelet consist either of M or E.

According to one embodiment, the smallest dimension of the final nanoplatelet comprises either an alternate of atomic layers of M and E.

According to one embodiment, the number of atomic layers of M in the final nanoplatelet is equal to one plus the number of atomic layer of E.

According to one embodiment, the shell is homogeneous thereby producing a final nanoplatelet.

According to one embodiment, the shell comprises a substantially identical thickness on each facet on the initial nanoplatelet.

In another embodiment of the invention, the nanoparticle of the invention may be further surrounded by a "coat" of an organic capping agent. The organic capping agent may be any number of materials, but has an affinity for the semiconductor surface. In general, the capping agent can be an isolated organic molecule, a polymer (or a monomer for a polymerization reaction), an inorganic complex, and an extended crystalline structure.

In one embodiment of the invention, examples of the nanoparticle of the invention include, but are not limited to, nanosheet of CdSe with a shell of CdS or ZnS (having a quantum yield superior to 80% and a narrow FHWM (less than 20 nm)), nanosheet of CdS coated with ZnS (CdS/Zns), nanosheet of CdSexS1-x (x being a decimal number from 0 to 1) coated with CdyZn1-yS (y being a decimal number from 0 to 1) (CdSeS/CdZnS), nanosheet of CdSexSe1-x (x being a decimal number from 0 to 1) coated with ZnS (CdSeS/ZnS), nanosheet of CdTe coated with CdyZn1-ySexS1-x ((x and y being independently a decimal number from 0 to 1) CdTe/CdZnSeS), nanosheet of CdS coated with ZnS doped with Manganese II or Copper II (CdS/ZnS:Mn or CdS/ZnS:Cu).

Preferably, the nanoparticle of the invention for use as a fluorophore, fluorescent agent or marker is selected in the group comprising CdSe/ZnS, CdSe/CdS, CdSe/CdS/ZnS, CdSe/ CdZnS/ZnS, CdSe/CdZnS, CdTe/CdS/CdZnS, CdS/ZnS.

The present invention relates to a process of growth of a shell on initial colloidal nanoplatelets.

According to one embodiment, the initial nanoplatelet is obtained by any method known from one skilled in the art.

According to one embodiment, the process of growth of a shell comprises the growth of an homogeneous shell on each facet of the initial colloidal nanoplatelet.

According to one embodiment, the process of growth of a core/shell nanoplatelets comprising a ME shell on initial colloidal nanoplatelets comprises the steps of injecting the initial colloidal nanoplatelets in a solvent at a temperature ranging from 200°C to 460°C and subsequently a precursor of E or M, wherein said precursor of E or M is injected slowly in order to control the shell growth rate; and wherein the precursor of respectively M or E is injected either in the solvent before injection of the initial colloidal nanoplatelets or in the mixture simultaneously with the precursor of respectively E or M.

According to one embodiment, the initial colloidal nanoplatelets are mixed with a fraction of the precursor's mixture before injection in the solvent.

According to one embodiment, the process of growth of a MxEy shell on initial colloidal nanoplatelets comprises the steps of injecting the initial colloidal nanoplatelets in a solvent at a temperature ranging from 200°C to 460°C and subsequently a precursor of E or M, wherein said precursor of E or M is injected slowly in order to control the shell growth rate; and wherein the precursor of respectively M or E is injected either in the solvent before injection of the initial colloidal nanoplatelets or in the mixture simultaneously with the precursor of respectively E or M; wherein x and y are independently a decimal number from 0 to 5.

According to one embodiment, the process of growth of core/shell nanoplatelets comprising a ME shell on initial colloidal nanoplatelets comprises the following steps:
- heating a solvent at a temperature ranging from 200°C to 460°C;
- injecting in the solvent the initial colloidal nanoplatelets;
- injecting slowly in the mixture the precursor of E and the precursor of M;
- recovering the core/shell structure in the form of nanoplatelets.

According to another embodiment, the process of growth of core/shell nanoplatelets comprising a ME shell on initial colloidal nanoplatelets comprises the following steps:
- heating a solvent at a temperature ranging from 200°C to 460°C;
- injecting a precursor of M in the solvent;
- injecting in the mixture the initial colloidal nanoplatelets;
- injecting slowly in the mixture the precursor of E;
- recovering the core/shell structure in the form of nanoplatelets.

According to another embodiment, the process of growth of core/shell nanoplatelets comprising a ME shell on initial colloidal nanoplatelets comprises the following steps:
- heating a solvent at a temperature ranging from 200°C to 460°C;
- injecting a precursor of E in the solvent;
- injecting in the mixture the initial colloidal nanoplatelets;
- injecting slowly in the mixture the precursor of M;
- recovering the core/shell structure in the form of nanoplatelets.

According to another embodiment, the process of growth of core/shell nanoplatelets comprising a ME shell on initial colloidal nanoplatelets comprises the following steps:
- heating a solvent at a temperature ranging from 200°C to 460°C;
- injecting in the solvent the initial colloidal nanoplatelets, optionally mixed with a fraction of the precursors mixture;
- injecting slowly in the mixture the precursor of E and the precursor of M;
- recovering the core/shell structure in the form of nanoplatelets.

Herein the term "fraction of the precursor's mixture" refers to a part of the total amount of precursors used in the reaction, i.e. from 0.001% to 50 %, preferably from 0.001% to 25%, more preferably from 0.01% to 10% of the total amount of the injected precursors mixture.

According to another embodiment, the process of growth of core/shell nanoplatelets comprising a ME shell on initial colloidal nanoplatelets comprises the following steps:
- providing a solvent and a precursor of M;
- heating the mixture of the solvent and the precursor of M at a temperature ranging from 200°C to 460°C;
- injecting in the mixture the initial colloidal nanoplatelets;
- injecting slowly in the mixture the precursor of E;
- recovering the core/shell structure in the form of nanoplatelets.

According to another embodiment, the process of growth of core/shell nanoplatelets comprising a ME shell on initial colloidal nanoplatelets comprises the following steps:
- providing a solvent and a precursor of E;
- heating the mixture of the solvent and the precursor of E at a temperature ranging from 200°C to 460°C;
- injecting in the mixture the initial colloidal nanoplatelets;
- injecting slowly in the mixture the precursor of M;
- recovering the core/shell structure in the form of nanoplatelets.

According to one embodiment, the initial colloidal nanoplatelets have a core/shell structure.

According to one embodiment, the process of growth of core/shell nanoplatelets comprising a ME shell on initial colloidal nanoplatelets further comprises the step of maintaining the mixture at a temperature ranging from 200°C to 460°C during a predetermined duration ranging from 1 to 180 minutes after the end of the injection of the second precursor.

According to one embodiment, the temperature of the annealing ranges from 200°C and 460°C, from 275°C to 365°C, from 300°C to 350°C or about 300°C.

According to one embodiment, the duration of the annealing ranges from 1 to 180 minutes, from 30 to 120 minutes, from 60 to 120 minutes or about 90 minutes.

According to one embodiment, the initial colloidal nanoplatelets are injected over a period of less than 10 minutes, less than 5 minutes, less than 1 minute, less than 30 seconds, less than 10 seconds, less than 5 seconds or less than 1 second. According to one embodiment, the initial colloidal nanoplatelets are injected at once.

According to one embodiment, the initial colloidal nanoplatelets are injected at a rate ranging from 1 mL/s to 1 L/s, from 1 mL/s to 100 mL/s, from 1 mL/s to 10 mL/s, from 2 to 8 mL/s or about 5 mL/s.

According to one embodiment, the injection of the precursor of E or the precursor of M of the shell is performed at a rate ranging from 0.1 to 30 mole/h/mole of M present in the initial nanoplatelet, preferably from 0.2 to 20 mole/h/mole of M present in the initial nanoplatelet, more preferably from 1 to 21 mole/h/mole of M present in the initial nanoplatelets.

According to one embodiment, the precursor of E or the precursor of M is injected slowly i.e. over a period ranging from 1 minute to 2 hours, from 1 minutes to 1 hour, from 5 to 30 minutes or from 10 to 20 minutes for each monolayer.

According to one embodiment, the precursor of E is injected slowly, i.e. at a rate ranging from 0.1 mL/h to 10 L/h, from 0.5 mL/h to 5 L/h or from 1 mL/h to 1 L/h.

According to one embodiment, the precursor of M is injected slowly, i.e. at a rate ranging from 0.1 mL/h to 10 L/h, from 0.5 mL/h to 5 L/h or from 1 mL/h to 1 L/h.

According to one embodiment, the precursor of E and the precursor of M are injected slowly in order to control the shell growth rate.

According to one embodiment wherein the precursor of M or the precursor of E is injected prior to the initial colloidal nanoplatelets, said precursor of M or said precursor of E is injected over a period of less than 30 seconds, less than 10 seconds, less than 5 seconds, less than 1 second. According to another embodiment wherein the precursor of M or the precursor of E is injected prior to the initial colloidal nanoplatelets, said precursor of M or said precursor of E is injected slowly, i.e. at a rate ranging from 0.1 mL/h to 10 L/h, from 0.5 mL/h to 5 L/h or from 1 mL/h to 1 L/h.

According to one embodiment, the precursor of M or the precursor of E injected prior to the initial colloidal nanoplatelets is injected faster than the precursor of M or the precursor of E injected after the initial colloidal nanoplatelets.

According to one embodiment, the injection's rate of at least one of the precursor of E and/or the precursor of M is chosen such that the growth rate of the shell is ranging from 1 nm per second to 0.1 nm per hour.

According to one embodiment, the growth process is performed at temperature ranging from 200°C to 460°C, from 275°C to 365°C, from 300°C to 350°C or about 300°C.

According to one embodiment, the reaction is performed under an inert atmosphere, preferably nitrogen or argon atmosphere.

According to one embodiment, the precursor of E is capable of reacting with the precursor of M to form a material with the general formula ME.

According to one embodiment, the precursor of the shell to be deposited is a precursor of a material MxEy, wherein:
M is Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is O, S, Se, Te, N, P, As, F, Cl, Br, I, or a mixture thereof,
and x and y are independently a decimal number from 0 to 5.

According to an embodiment, the precursor of the shell to be deposited is a material MxEy comprising cationic element M and anionic element E in stoichiometric ratio, said stoichiometric ratio being characterized by values of x and y corresponding to absolute values of mean oxidation number of elements E and M respectively.

According to a preferred embodiment, the precusrsor of the shell to be deposited is a precursor of a material MxEy wherein:
M is selected from group Ib, IIa, IIb, IIIa, IIIb, IVa, IVb, Vb, VIb, VIIb, VIII or mixtures thereof;
E is selected from group Va, VIa, VIIa or mixtures thereof; and
x and y are independently a decimal number from 0 to 5.

According to one embodiment, the precursor of the shell to be deposited is a precursor of a compound of group IIb-VIa, group IVa-VIa, group Ib-IIIa-VIa, group IIb-IVa-Va, group Ib-VIa, group VIII-VIa, group IIb-Va, group IIIa-VIa, group IVb-VIa, group IIa-VIa, group IIIa-Va, group IIIa-VIa, group VIb-VIa, or group Va-VIa.

According to one embodiment, the precursor of the shell to be deposited is a precursor of a material chosen among CdS, CdSe, CdTe, CdO, Cd₃P₂, Cd₃As₂, ZnS, ZnSe, ZnO, ZnTe, Zn₃P₂, Zn3As2, HgS, HgSe, HgTe, HgO, GeS, GeSe, GeTe, SnS, SnS₂, SnSe₂, SnSe, SnTe, PbS, PbSe, PbTe, GeS₂, GeSe₂, CuInS₂, CuInSe₂, CuS, Cu₂S, Ag₂S, Ag₂Se, Ag₂Te AgInS₂, AgInSe₂, FeS, FeS₂, FeO, Fe₂O₃, Fe₃O₄, Al₂O₃, TiO₂, MgO, MgS, MgSe, MgTe, AlN, AlP, AlAs, AlSb, GaN, GaP, GaAs, GaSb, InN, InP, InAs, InSb, In₂S₃,TlN, TlP, TlAs, TISb, Bi₂S₃, Bi₂Se₃, Bi₂Te₃, MoS₂, WS₂, VO₂ or a mixture thereof.

According to a preferred embodiment, the precursor of the shell to be deposited is a precursor of a material selected from the group consisting of CdS, CdSe, CdSSe, CdTe, ZnO, ZnS, ZnSe, ZnTe, PbS, PbSe, PbTe, CuInS₂, CuInSe₂, AgInS₂, AgInSe₂, CuS, Cu₂S, Ag₂S, Ag₂Se, Ag₂Te, FeS, FeS₂, PdS, Pd₄S, WS₂ or a mixture thereof.

According to one embodiment, if E is a chalcogenide, the precursor of E is a compound containing the chalcogenide at the -2 oxidation state. According to one embodiment, if E is a chalcogenide, the precursor of E is formed in situ by reaction of a reducing agent with a compound containing E at the 0 oxidation state or at a strictly positive oxidation state.

According to one embodiment, if E is sulfur, the precursor of E is a thiol. According to one embodiment, if E is sulfur, the precursor of E is dodecanethiol or octanethiol. According to one embodiment, if E is sulfur, the precursor of E is a salt containing S²⁻ sulfide ions. According to one embodiment, if E is sulfur, the precursor of E comprises bis(trimethylsilyl) sulfide (TMS₂S) or hydrogen sulfide (H₂S) or sodium hydrogen sulfide (NaSH) or sodium sulfide (Na₂S) or ammonium sulfide (S(NH₄)₂) or thiourea or thioacetamide. According to one embodiment, if E is sulfur, the precursor of E is sulfur dissolved in a suitable solvent. According to one embodiment, if E is sulfur, the precursor of E is sulfur dissolved in 1-octadecene. According to one embodiment, if E is sulfur, the precursor of E is sulfur dissolved in a phosphine. According to one embodiment, if E is sulfur, the precursor of E is sulfur dissolved in trioctylphosphine or tributylphosphine. According to one embodiment, if E is sulfur, the precursor of E is sulfur dissolved in an amine. According to one embodiment, if E is sulfur, the precursor of E is sulfur dissolved in oleylamine. According to one embodiment, if E is sulfur, the precursor of E is sulfur powder dispersed in a solvent. According to one embodiment, if E is sulfur, the precursor of E is sulfur powder dispersed in 1-octadecene.

According to one embodiment, if E is selenium, the precursor of E comprises a salt containing Se²⁻ selenide ions. According to one embodiment, the precursor of E comprises bis(trimethylsilyl) selenide (TMS₂Se) or hydrogen selenide (H₂Se) or sodium selenide (Na₂Se) or sodium hydrogen selenide (NaSeH) or sodium selenosulfate (Na₂SeSO₃) or selenourea. According to one embodiment, if E is selenium, the precursor of E is a selenol. According to one embodiment, if E is selenium, the precursor of E is a diselenide, such as Diphenyl diselenide. According to one embodiment, if E is selenium, the precursor of E is selenium dissolved in a suitable solvent. According to one embodiment, if E is selenium, the precursor of E is selenium dissolved in 1-octadecene. According to one embodiment, if E is selenium, the precursor of E is selenium dissolved in a phosphine. According to one embodiment, if E is selenium, the precursor of E is selenium dissolved in trioctylphosphine or tributylphosphine. According to one embodiment, if E is selenium, the precursor of E is selenium dissolved in an amine. According to one embodiment, if E is selenium, the precursor of E is selenium dissolved in an amine and thiol mixture. According to one embodiment, if E is selenium, the precursor of E is selenium powder dispersed in a solvent. According to one embodiment, if E is selenium, the precursor of E is selenium powder dispersed in 1-octadecene.

According to one embodiment, if E is tellurium, the precursor of E is as salt containing Te²⁻ telluride ions. According to one embodiment, if E is tellurium, the precursor of E comprises bis(trimethylsilyl) telluride (TMS₂Te) or hydrogen telluride (H₂Te) or sodium telluride (Na₂Te) or sodium hydrogen telluride (NaTeH) or sodium tellurosulfate (Na₂TeSO₃) or tellurourea. According to one embodiment, if E is tellurium, the precursor of E is tellurium dissolved in a suitable solvent. According to one embodiment, if E is tellurium, the precursor of E is tellurium dissolved a phosphine. According to one embodiment, if E is tellurium, the precursor of E is tellurium dissolved in trioctylphosphine or tributylphosphine.

According to one embodiment, if E is oxygen, the precursor of E is the hydroxide ion (HO⁻). According to one embodiment, if E is oxygen the precursor of E is a solution of sodium hydroxide (NaOH) or of potassium hydroxide (KOH) or of tetramethylammonium hydroxide (TMAOH). According to one embodiment, if E is oxygen, the precursor of E is generated in-situ by condensation between an amine and a carboxylic acid. According to one embodiment, if E is oxygen, the precursor of E is generated in-situ by condensation of two carboxylic acids.

According to one embodiment, if E is phosphorus, the precursor of E comprises phosphorus at the -3 oxidation state. According to one embodiment, the precursor of E comprises tris(trimethylsilyl) phosphine (TMS₃P) or phosphine (PH₃) or white phosphorus (P₄) or phosphorus trichloride (PCl₃). According to one embodiment, the precursor of E comprises a tris(dialkylamino)phosphine for example tris(dimethylamino)phosphine ((Me₂N)₃P) or tris(diethylamino)phosphine ((Et₂N)₃P). According to one embodiment, the precursor of E comprises a trialkylphosphine for example trioctylphosphine or tributylphosphine or triphenylphosphine.

According to one embodiment, if M is a metal, the precursor of M is a compound containing the metal at positive or 0 oxidation state. According to one embodiment, if M is a metal, the precursor of M comprises a metallic salt. In one embodiment, the metallic salt is a carboxylate of M, or a chloride of M, or a bromide of M, or an iodide of M, or a nitrate of M, or a sulfate of M, or a thiolate of M. According to one embodiment, the shell comprises a metal.

According to one embodiment, the shell to be deposited comprises a chalcogenide, a phosphide, a nitride, an arsenide or an oxide.

According to one embodiment, the initial nanosheet is dispersed in a solvent. According to one embodiment, the solvent is organic, preferably apolar or weakly polar. According to one embodiment, the solvent is a supercritical fluid or an ionic fluid. According to one embodiment, the solvent is selected from distilled water, methanol, ethanol, isopropanol, butanol, chloroform, acetone, hexane, tetrahydrofuran, dimethylsulfoxide, toluene, octadecene, squalane, trioctylamine, oleylamine, hexadecylamine, octadecylamine, squalene, and/or dimethylformamide.

According to one embodiment, the shell comprises an additional element in minor quantities. The term "minor quantities" refers herein to quantities ranging from 0.0001% to 10% molar, preferably from 0.001% to 10% molar.

According to one embodiment, the shell comprises a transition metal or a lanthanide in minor quantities. The term "minor quantities" refers herein to quantities ranging from 0.0001 % to 10% molar, preferably from 0.001 % to 10% molar.

According to one embodiment, the shell comprises in minor quantities an element inducing an excess or a defect of electrons compared to the sole film. The term "minor quantities" refers herein to quantities ranging from 0.0001% to 10% molar, preferably from 0.001% to 10% molar.

According to one embodiment, a reducing agent is introduced at the same time as at least one of the precursor of M and/or E. In one embodiment, the reducing agent comprises a hydride. Said hydride may be selected from sodium tetrahydroborate (NaBH4); sodium hydride (NaH), lithium tetrahydroaluminate (LiAlH4), diisobutylaluminum hydride (DIBALH). In one embodiment, the reducing agent comprises dihydrogen.

According to one embodiment, a stabilizing compound capable of stabilizing the final nanoplatelet is introduced in the solvent.

According to one embodiment, a stabilizing compound capable of stabilizing the final nanoplatelet is introduced in anyone of the precursor solutions.

According to one embodiment, the stabilizing compound of the final nanoplatelet comprises an organic ligand. Said organic ligand may comprise a carboxylic acid, a thiol, an amine, a phosphine, an amide, an ester, a pyridine, an imidazole and/or an alcohol. According to one embodiment, the stabilizing compound of the final nanoplatelet is an ion. Said ion comprises a quaternary ammonium.

According to one embodiment, the initial nanosheet is fixed on a least one substrate.

According to one embodiment, the fixation of the initial nanosheet on said substrate is performed by adsorption or chemical coupling.

According to one embodiment, said substrate is chosen among silica SiO₂, aluminum oxide Al₂O₃, indium-tin oxide ITO, fluorine-doped tin oxide FTO, titanium oxide TiO₂, gold, silver, nickel, molybdenum, aluminum, silicium, germanium, silicon carbide SiC, graphene and cellulose.

According to one embodiment, said substrate comprises a polymer.

According to one embodiment, the excess of precursors is discarded after the reaction.

According to one embodiment, the final nanoplatelet obtained after reaction of the precursors on the initial nanosheets is purified. Said purification is performed by flocculation and/or precipitation and/or filtration; such as for example successive precipitation in ethanol.

The present invention also relates to a population of semiconductor nanoplatelets, each member of the population comprising a nanoplatelet core including a first semiconductor material and at least one shell including a second semiconductor material on the surface of the nanoplatelet core, wherein after ligand exchange reaction the population exhibits a quantum yield decrease of less than 50%.

According to one embodiment, the population of semiconductor nanoplatelets of the present invention exhibit, after ligand exchange, a quantum yield decrease of less than 50%, less than 40%, less than 30%, less than 25%, less than 20%, less than 15% or less than 10%.

Especially, according to one embodiment, after transfer into an aqueous solution by ligand exchange reaction, the quantum yield of the population of nanoplatelets according to the present invention decrease of less than 50%, less than 40%, less than 30%, less than 25%, less than 20%, less than 15% or less than 10%.

According to one embodiment, the ligand is an organic ligand with a carbonated chain length between 1 and 30 carbons.

According to one embodiment, the ligand is a polymer.

According to one embodiment, the ligand is a hydrosoluble polymer.

According to one embodiment, the selected ligand may comprise a carboxylic acid, a thiol, an amine, a phosphine, a phosphine oxide, an amide, an ester, a pyridine, an imidazole and/or an alcohol.

According to one embodiment, the ligand is selected from myristic acid, stearic acid, palmitic acid, oleic acid, behenic acid, dodecanethiol, oleylamine, 3-mercaptopropionic acid.

According to one embodiment, the selected ligand may be any number of materials, but has an affinity for the semiconductor surface. In general, the capping agent can be an isolated organic molecule, a polymer (or a monomer for a polymerization reaction), an inorganic complex, and an extended crystalline structure.

According to one embodiment, the ligand exchange procedure comprises the step of treating a solution of nanoplatelets according to the invention with a ligand.

The present invention also relates to a population of semiconductor nanoplatelets wherein the population exhibits stable fluorescence quantum efficiency over time. According to one embodiment, the population of nanoplatelets, wherein each member of the population comprising a nanoplatelet core including a first semiconductor material and a shell including a second semiconductor material on the surface of the nanoplatelet core, exhibits fluorescence quantum efficiency decrease of less than 50%, less than 40%, less than 30% after one hour under light illumination.

According to one embodiment, the light illumination is provided by blue or UV light source such as laser, diode or Xenon Arc Lamp.

According to one embodiment, the photon flux of the illumination is comprised between 1mW.cm⁻² and 100W.cm⁻² and more preferably between 10mW.cm⁻² and 50W.cm⁻², and even more preferably between 10mW.cm⁻² and 10W.cm⁻².

According to one embodiment, the population of nanoplatelets, wherein each member of the population comprising a nanoplatelet core including a first semiconductor material and a shell including a second semiconductor material on the surface of the nanoplatelet core, exhibits fluorescence quantum efficiency decrease of less than 80%, less than 70%, less than 60%, less than 50%, less than 40%, less than 30%, less than 20% or less than 15% after 2 months after a ligand exchange.

According to one embodiment, the semiconductor nanoplatelets of the invention exhibit enhanced stability in time compared to quantum dots and nanoplatelets of the prior art.

According to one embodiment, the semiconductor nanoplatelets of the invention exhibit enhanced stability in temperature compared to quantum dots and nanoplatelets of the prior art.

According to one embodiment, the core/shell nanoplatelets according to the present invention exhibit stable fluorescence quantum efficiency in temperature. Especially, according to one embodiment, the population of semiconductor nanoplatelets according to the invention exhibits fluorescence quantum efficiency at 100 °C or above that is at least 50%, at least 60%, at least 70%, at least 80%, or at least 90% of the fluorescence quantum efficiency of the population at 20°C. According to one embodiment, the temperature is in a range from 100°C to 250°C, from 100°C to 200°C, from 110°C to 160°C or about 140°C. According to one embodiment, the population of semiconductor nanoplatelets according to the invention exhibits fluorescence quantum efficiency at 200°C that is at least 50%, at least 60%, at least 70%, at least 80% or at least 90% of the fluorescence quantum efficiency of the population at 20°C.

According to one embodiment, the population of nanoplatelets according to the present invention exhibit emission spectra with a full width half maximum lower than 50, 40, 30, 25 nm or 20 nm.

The present invention also relates to a population of fluorescent colloidal nanoplatelets, each member of the population comprising a nanoplatelet core including a first semiconductor material and a shell including a second semiconductor material on the surface of the nanoplatelet core, wherein at least 40% of the nanoplatelets of the population are continuously emissive for a period of at least one minute.

According to one embodiment, at least 40%, at least 50%, at least 60%, at least 70%, at least 75%, at last 80% or at least 90% of the nanoplatelets of the population are continuously emissive for a period of at least one minute.

According to one embodiment, the shell of the nanoplatelets of the population has a thickness of at least 3 nm, at least 5 nm, at least 5.5 nm, at least 6 nm, at least 7 nm, at least 8 nm, at least 8.5 nm, at least 9 nm, at least 10 nm.

According to one embodiment, as depicted in figure 10, at least 40% of a population of core/shell nanoplatelets having a shell with a thickness of at least 3 nm is continuously emissive for a period of at least one minute. According to one embodiment, as depicted in figure 12, at least 85% of a population of core/shell nanoplatelets having a shell with a thickness of at least 5.5 nm is continuously emissive for a period of at least one minute. According to one embodiment, as depicted in figure 15, at least 90% of a population of core/shell nanoplatelets having a shell with a thickness of at least 8 nm is continuously emissive for a period of at least one minute.

Another object of the invention is the use of core/shell nanoplatelets according to the invention for use as fluorophore, fluorescent agent or marker.

In one embodiment, the surface of the core/shell nanoplatelet of the invention can be modified to produce a fluorophore that can be coupled to a variety of biological molecules or substrates by techniques well known in the art.

In another aspect, the present invention relates to chemical and biological assays which use the nanoplatelet fluorophore of the invention as detectable luminescent labels to detect the presence or amount of one or more molecules, biomolecules or analyte, as well as to detect biological interactions, biological processes, alterations in biological processes, or alterations in the structure of a chemical or biological compound.

The nanoplatelet fluorophore of the invention can be used to detect or track a single target. Additionally, a population of fluorophores of the invention may be used for either simultaneous detection of multiple targets or to detect particular compounds and/or items of interest in, e. g., a library of compounds. For example, compositions of nanoplatelets fluorophores of the invention comprising one or more particle size distributions having characteristic spectral emissions may be used as "barcodes" in assays to either track the location or source of a particular item of interest or to identify a particular item of interest. The fluorophores of the invention used in such a "barcoding" scheme can be tuned to a desired wavelength to produce a characteristic spectral emission by changing their composition and size, or size distribution. Additionally, the fluorescence quantum efficiency of the emission at a particular characteristic wavelength can also be varied, thus enabling the use of binary or higher order encoding schemes.

The information encoded by the nanoplatelets fluorophores of the invention can be spectroscopically decoded, thus providing the location and/or identity of the particular item or component of interest. In another example, composition of nanoplatelets fluorophores of the invention having characteristic fluorescence lifetimes may be used as "barcodes" in assays to either track the location or source of a particular item of interest or to identify a particular item of interest. The fluorophores of the invention used in such a "barcoding" scheme can be tuned to a desired fluorescence lifetime to produce a characteristic fluorescence lifetime by changing their composition and size, or size distribution.

The nanoplatelets fluorophores of the invention can be used to detect the presence and/or amount of a biological moiety, e. g., a biological target analyte; the structure, composition, and conformation of a biological molecule; the localization of a biological moiety, e. g., a biological target analyte in an environment; interactions of biological molecules; alterations in structures of biological compounds; and/or alterations in biological processes.

Thus, it is readily apparent that the nanoplatelets fluorophores of the invention find use in a variety of assays where other, less reliable, labeling methods have typically been used, including, without limitation, fluorescence microscopy, histology, cytology, pathology, flow cytometry, western blotting, Fluorescence Resonance Energy Transfer (FRET), immunocytochemistry, Fluorescence In Situ Hybridization (FISH) and other nucleic acid hybridization assays, signal amplification assays, DNA and protein sequencing, immunoassays such as competitive binding assays and ELISAs, immunohistochemical analysis, protein and nucleic acid separation, homogeneous assays, multiplexing, high throughput screening, chromosome karyotyping, and the like.

Another object of the invention is a composition comprising the nanoplatelet fluorophore of the invention associated with a specific-binding component, such that the composition can detect the presence and/or amounts of biological and chemical compounds, detect interactions in biological systems, detect biological processes, detect alterations in biological processes, or detect alterations in the structure of biological compounds. Without limitation, the fluorophore conjugates comprise any component linked to the nanoplatelet fluorophore of the invention that can interact with a biological target, to detect biological processes, or reactions, as well as alter biological molecules or processes.

Another object of the invention is a fluorophore conjugate resulting from attachment of the nanoplatelet fluorophore of the invention and a component as defined here after.

In one embodiment, said component may be chosen among antigens, steroids, vitamins, drugs, haptens, metabolites, toxins, environmental pollutants, amino acids, peptides, proteins, nucleic acids, nucleic acid polymers, carbohydrates, lipids, and polymers. In another embodiment, said component may be chosen among an amino acid, peptide, protein, antibody, polysaccharide, nucleotide, nucleoside, oligonucleotide, nucleic acid, hapten, a psoralen, drug, a hormone, lipid, phospholipid, lipoprotein, lipopolysaccharide, liposome, lipophilic polymer, a synthetic polymer, polymeric microparticle, biological cell, a virus and combinations thereof.

In one embodiment of the invention, said component is labeled with a plurality of fluorophores of the invention, which may be the same or different.

In one embodiment, said component comprises an amino acid (including those that are protected or are substituted by phosphates, carbohydrates, or C1 to C22 carboxylic acids), or a polymer of amino acids such as a peptide or protein. Preferred conjugates of peptides contain at least five amino acids, more preferably 5 to 36 amino acids. Preferred peptides include, but are not limited to, neuropeptides, cytokines, toxins, protease substrates, and protein kinase substrates. Also preferred are peptides that serve as organelle localization peptides, that is, peptides that serve to target the conjugated compound for localization within a particular cellular substructure by cellular transport mechanisms. Preferred protein conjugates include enzymes, antibodies, lectins, glycoproteins, histones, albumins, lipoproteins, avidin, streptavidin, protein A, protein G, phycobiliproteins and other fluorescent proteins, hormones, toxins and growth factors. Typically, the conjugated protein is an antibody, an antibody fragment, avidin, streptavidin, a toxin, a lectin, or a growth factor.

In another embodiment, said component comprises a nucleic acid base, nucleoside, nucleotide or a nucleic acid polymer. Preferred nucleic acid polymer conjugates are single- or multi-stranded, natural or synthetic DNA or RNA oligonucleotides, or DNA/RNA hybrids, or incorporating an unusual linker such as morpholine derivatized phosphates, or peptide nucleic acids such as N-(2-aminoethyl)glycine units, where the nucleic acid contains fewer than 50 nucleotides, more typically fewer than 25 nucleotides.

In another embodiment, said component comprises a carbohydrate or polyol that is typically a polysaccharide, such as dextran, FICOLL, heparin, glycogen, amylopectin, mannan, inulin, starch, agarose and cellulose, or is a polymer such as a poly(ethylene glycol).

In another embodiment, said component comprises a lipid (typically having 6-25 carbons), including glycolipids, phospholipids, and sphingolipids. Alternatively, said molecule or molecular complex comprises a lipid vesicle, such as a liposome, or is a lipoprotein. Some lipophilic substituents are useful for facilitating transport of the fluorophore of the invention into cells or cellular organelles.

In another embodiment, said component includes polymers, polymeric particles, polymeric microparticles including magnetic and non-magnetic microspheres, polymeric membranes, conducting and non-conducting metals and non-metals, and glass and plastic surfaces and particles. Conjugates are typically prepared by chemical modification of a polymer that contains functional groups with suitable chemical reactivity. The conjugated polymer may be organic or inorganic, natural or synthetic. In a preferred embodiment, the present compounds are conjugated to a polymer matrix, such as a polymeric particle or membrane, including membranes suitable for blot assays for nucleic acids or proteins. In another embodiment, said molecule or molecular complex comprises a glass or silica, which may be formed into an optical fiber or other structure. In another embodiment, said molecule or molecular complex comprises a poly(ethylene glycol), a poly(acrylate) or a poly(acrylamide).

Alternatively, the conjugates of the present invention are conjugates of cells, cellular systems, cellular fragments, or subcellular particles. Examples of this type of conjugated material include virus particles, bacterial particles, virus components, biological cells (such as animal cells, plant cells, bacteria, or yeast), or cellular components. Examples of cellular components that can be labeled, or whose constituent molecules can be labeled, include but are not limited to lysosomes, endosomes, cytoplasm, nuclei, histones, mitochondria, Golgi apparatus, endoplasmic reticulum and vacuoles.

The fluorophore conjugates can be made using techniques known in the art. For example, moieties such as Tri octyl phosphine oxide (TOPO) and tri octyl phosphine (TOP), may be readily displaced and replaced with other functional moieties, including, but not limited to carboxylic acids, amines, aldehydes, and styrene to name a few. The person skilled in the art will realize that factors relevant to the success of a particular displacement reaction include the concentration of the replacement moiety, temperature and reactivity.

The ability to utilize a general displacement reaction to modify selectively the surface functionality of the fluorophore of the invention enables functionalization for specific uses. For example, because detection of biological compounds is most preferably carried out in aqueous media, a preferred embodiment of the present invention utilizes nanoplatelets fluorophores of the invention that are solubilized in water. In the case of water-soluble fluorophores, the outer layer includes a compound having at least one linking moiety that attaches to the surface of the nanoplatelet and that terminates in at least one hydrophilic moiety. The linking and hydrophilic moieties are spanned by a hydrophobic region sufficient to prevent charge transfer across the region. The hydrophobic region also provides a "pseudohydrophobic" environment for the fluorophore and thereby shields it from aqueous surroundings. The hydrophilic moiety may be a polar or charged (positive or negative) group. The polarity or charge of the group provides the necessary hydrophilic interactions with water to provide stable solutions or suspensions of the nanoplatelets of the invention. Exemplary hydrophilic groups include polar groups such as hydroxides (-OH), amines, polyethers, such as polyethylene glycol and the like, as well as charged groups, such as carboxylates (-CO2⁻), sulfonates (-SO3⁻), phosphonates (-PO4²⁻), nitrates, ammonium salts (NH4⁺), and the like. A water-solubilizing layer is found at the outer surface of the overcoating layer. Methods for rendering the fluorophore of the invention water-soluble are known in the art.

Additional modifications can also be made such that the nanoplatelet fluorophore of the invention can be associated with almost any solid support. A solid support, for the purposes of this invention, is defined as an insoluble material to which compounds are attached during a synthesis sequence, screening, immunoassays, etc. The use of a solid support is particularly advantageous for the synthesis of libraries because the isolation of support-bound reaction products can be accomplished simply by washing away reagents from the support-bound material and therefore the reaction can be driven to completion by the use of excess reagents. A solid support can be any material that is an insoluble matrix and can have a rigid or semi-rigid surface. Exemplary solid supports include but are not limited to pellets, disks, capillaries, hollow fibers, needles, pins, solid fibers, cellulose beads, pore-glass beads, silica gels, polystyrene beads optionally cross-linked with divinylbenzene, grafted co-poly beads, polyacrylamide beads, latex beads, dimethylacrylamide beads optionally crosslinked with N-N'-bis acryloylethylenediamine, and glass particles coated with a hydrophobic polymer.

For example, the fluorophore of the invention can readily be functionalized to create styrene or acrylate moieties, thus enabling its incorporation into polystyrene, polyacrylate or other polymers such as polyimide, polyacrylamide, polyethylene, polyvinyl, polydiacetylene, polyphenylene-vinylene, polypeptide, polysaccharide, polysulfone, polypyrrole, polyimidazole, polythiophene, polyether, epoxies, silica glass, silica gel, siloxane, polyphosphate, hydrogel, agarose, cellulose, and the like.

Examples of fluorophore conjugate include, but are not limited to, fluorophore streptavidine conjugate, mouse IgG2a fluorophore conjugate, fluorophore anti-fluorescein conjugate, CD2 mAb (monoclonal antibody) fluorophore conjugate, CD3 mAb fluorophore conjugate, CD4 mAb fluorophore conjugate, CD8 mAb fluorophore conjugate, CD 14 mAb fluorophore conjugate, CD 19 mAb fluorophore conjugate, CD20 mAb fluorophore conjugate, CD25 mAb fluorophore conjugate, CD27 mAb fluorophore conjugate, CD45 mAb fluorophore conjugate, CD45R mAb fluorophore conjugate, CD45RA mAb fluorophore conjugate, CD56 mAb fluorophore conjugate, HLA DR mAb fluorophore conjugate, fluorophore donkey anti-goat IgG conjugate, fluorophore donkey anti-mouse IgG conjugate, fluorophore donkey anti-rabbit IgG conjugate, fluorophore goat F(ab')2 anti-mouse IgG, fluorophore goat F(ab')2 anti-rabbit IgG, fluorophore wheat germ agglutinin (WGA) conjugate.

Examples of nanoplatelet fluorophore functionalized ready to be used for conjugation include, but are not limited to, carboxylate functionalized fluorophore and amino (PEG) fluorophore.

Another object of the invention is the use of a nanoplatelet fluorophore or a fluorophore conjugate of the invention in a detection system, wherein said detection system includes, but is not limited to, an affinity assay, fluorescent staining, flow cytometry, nucleic acid sequencing, nucleic acid hybridization, nucleic acid synthesis or amplification, or molecular sorting.

Another object of the invention is a method for detecting an analyte in a sample, preferably a biological sample, said method comprising:
(a) contacting said sample with a conjugate as defined here above, wherein the component is a binding partner for said analyte;
(b) incubating said conjugate with said sample for a sufficient amount of time for said analyte and said component to interact, thereby forming a fluorescent analyte; and
(c) illuminating said fluorescent analyte with an appropriate wavelength, whereby the presence of said analyte is determined in said sample.

At any time after or during an assay or staining procedure, the sample is illuminated with a wavelength of light that results in a detectable optical response, and observed with a means for detecting the optical response. The nanoplatelets fluorophores of the invention are detected upon illumination, such as by an ultraviolet or visible wavelength emission lamp, an arc lamp, or a laser. Selected equipment that is useful for illuminating the fluorophore conjugates of the invention includes, but is not limited to, hand-held ultraviolet lamps, mercury arc lamps, xenon lamps, argon lasers, laser diodes, and YAG lasers. These illumination sources are optionally integrated into laser scanners, fluorescence microplate readers, standard or mini fluorometers, or chromatographic detectors. This fluorescence emission is optionally detected by visual inspection, or by use of any of the following devices: CCD cameras, video cameras, photographic film, laser scanning devices, fluorometers, photodiodes, quantum counters, epifluorescence microscopes, scanning microscopes, flow cytometers, fluorescence microplate readers, or by means for amplifying the signal such as photomultiplier tubes. Where the sample is examined using an instrument such as a flow cytometer, a fluorescence microscope or a fluorometer, the instrument is optionally used to distinguish and discriminate between distincts fluorophores with detectably different optical properties, typically by distinguishing the fluorescence response of one fluorophore conjugate from another one. Where the sample is examined using a flow cytometer, examination of the sample optionally includes isolation of particles within the sample based on the fluorescence response of the fluorophore by using a sorting device.

A detectable optical response means a change in, or occurrence of, a parameter in a test system that is capable of being perceived, either by direct observation or instrumentally. Such detectable responses include the change in, or appearance of, color, fluorescence, reflectance, chemiluminescence, light polarization, light scattering, or x-ray scattering. Typically the detectable response is a change in fluorescence, such as a change in the quantum efficiency, excitation or emission wavelength distribution of fluorescence, fluorescence lifetime, fluorescence polarization, or a combination thereof. The detectable optical response may occur throughout the sample or in a localized portion of the sample.

The presence or absence of the optical response after the elapsed time is indicative of one or more characteristic of the sample. Comparison of the degree of staining with a standard or expected response can be used to determine whether and to what degree the sample possesses a given characteristic.

In another embodiment, the nanoplatelets fluorophores or the fluorophore conjugates of the invention may be used in a multiplex assay for detecting one or more species in a mixture.

As used herein, the term "multiplex assay" refers to an assay in which fluorescence from two or more fluorophores is detected, or in which fluorescence energy transfer between two or more fluorophores and one or more quencher is detected.

Another object of the invention is a method for detecting multiple analytes in a sample, preferably a biological sample, said method comprising:
(a) contacting said sample with multiple conjugates as defined here above, wherein each component is a binding partner for one analyte and wherein each fluorophore presents a different fluorescent emission;
(b) incubating said conjugates with said sample for a sufficient amount of time for said analytes and said components to interact, thereby forming multiple fluorescent analytes; and
(c) illuminating said fluorescent analytes with an appropriate wavelength, whereby the presence of said analytes is determined in said sample.

Another object of the invention is a kit for detection of at least one analyte in a sample, wherein said kit comprises at least one nanoplatelet fluorophore as defined here above or at least one fluorophore conjugate as defined here above.

The nanoplatelets fluorophores of the invention can be used in the following fluorescence detection methods: FACS, multicolor optical coding of cells, microarrays, immunochemistry, multiplex FISH, fixed cell or tissue imaging.

The nanoplatelets fluorophores of the invention can also be used as biosensors: tagged antibodies, FRET sensors, encoded multiplexed microbeads.

Another use for the nanoplatelets fluorophores of the invention is in vivo animal imaging (cells, tissues, organs, tumors) for example in the context of photo-induced therapy, optical surgical aid or pharmacokinetic determination of therapeutic agents.
The fluorophores of the invention can also be used for ex vivo live cells imaging.

Other characteristics and advantages of the nanoplatelets according to the invention will appear after reading the examples given after as purely illustrative means.

### BRIEF DESCRIPTION OF THE DRAWINGS

**Figure 1** shows a) an absorption spectrum and b) an emission spectrum of CdSe/CdS core/shell nanoplatelets according to the present invention.
**Figure 2A** and **2B** show TEM images of CdSe/CdS core/shell nanoplatelets with a shell thickness of 1.5 nm according to the present invention.

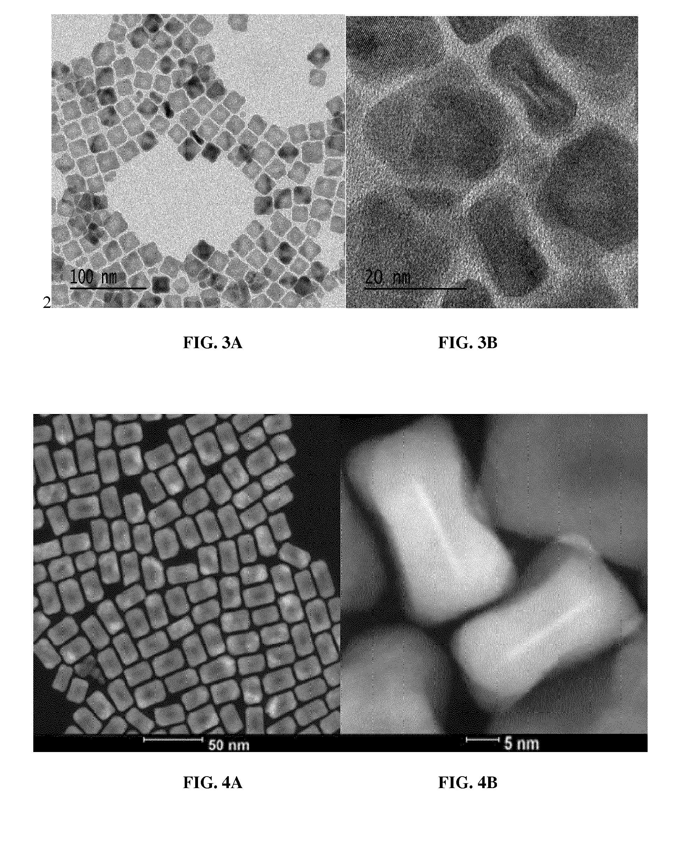

**Figure 3A** and **3B** show TEM images of CdSe/CdS core/shell nanoplatelets with a shell thickness of 3 nm according to the present invention.
**Figure 4A** and **4B** show TEM images of CdSe/CdS core/shell nanoplatelets with a shell thickness of 5.5 nm according to the present invention.
**Figure 5** shows a) an absorption spectrum and b) an emission spectrum of CdSe/CdZnS core/shell nanoplatelets according to the present invention.
**Figure 6** shows TEM images of CdSe/CdZnS core/shell nanoplatelets according to the present invention.

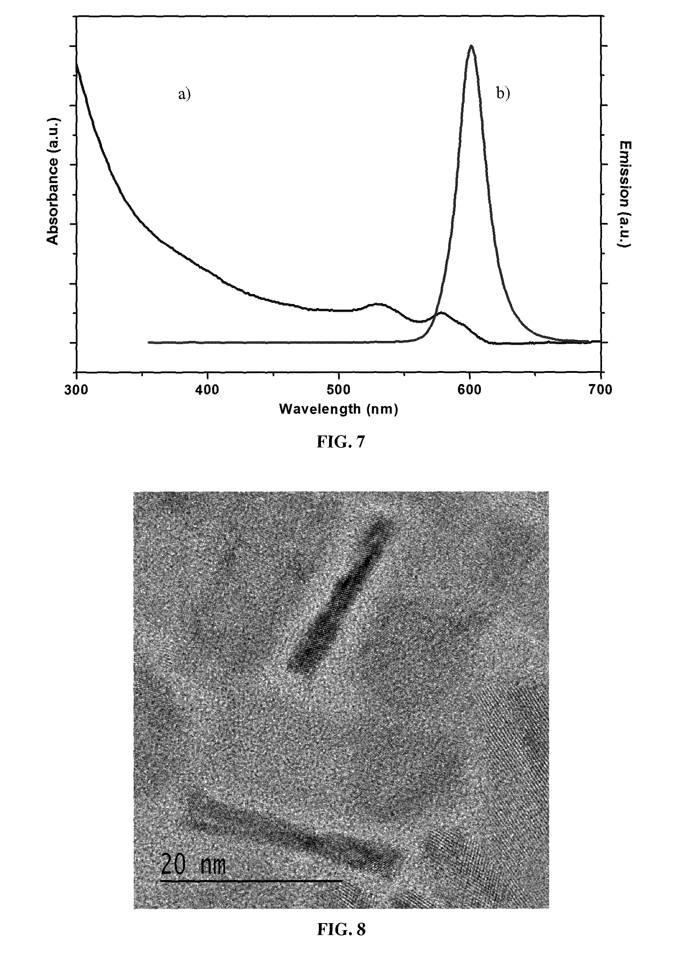

**Figure 7** shows a) an absorption spectrum and b) an emission spectrum of CdSe/ZnS core/shell nanoplatelets according to the present invention.
**Figure 8** shows TEM images of CdSe/ZnS core/shell nanoplatelets according to the present invention.

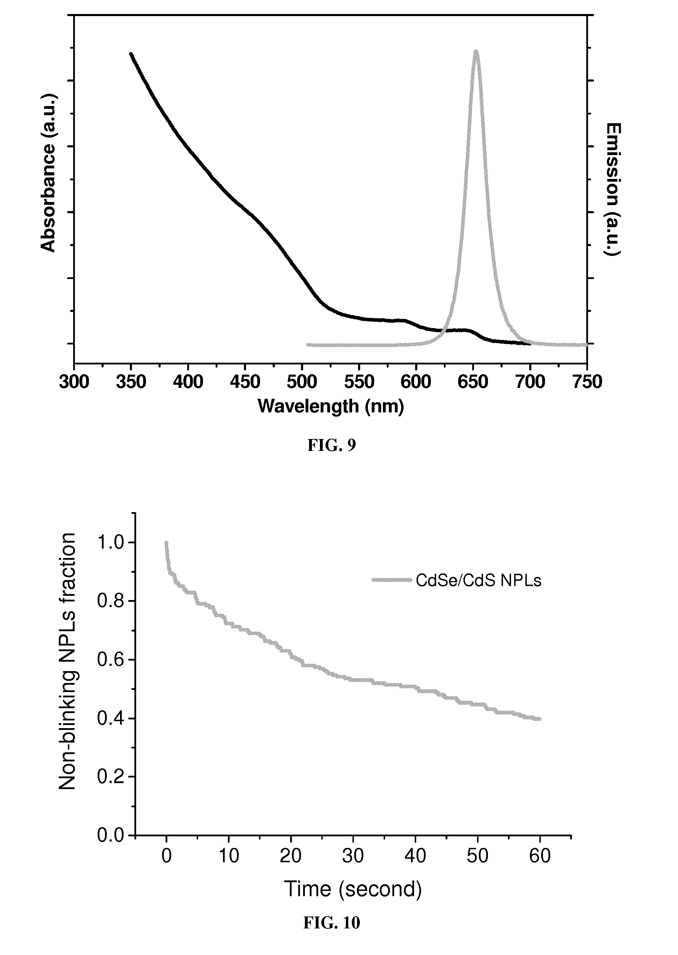

**Figure 9** shows the evolution of the absorption spectrum (black) and the emission spectrum (grey) of CdSe/CdZnS core/shell nanoplatelets according to the present invention after ligand exchange with a hydrosoluble polymer.
**Figure 10** shows the non-blinking fraction of CdSe/CdS core/shell nanoplatelets with a shell thickness of 3 nm according to the present invention (i.e. the fraction of continuously emissive nanoplatelets).

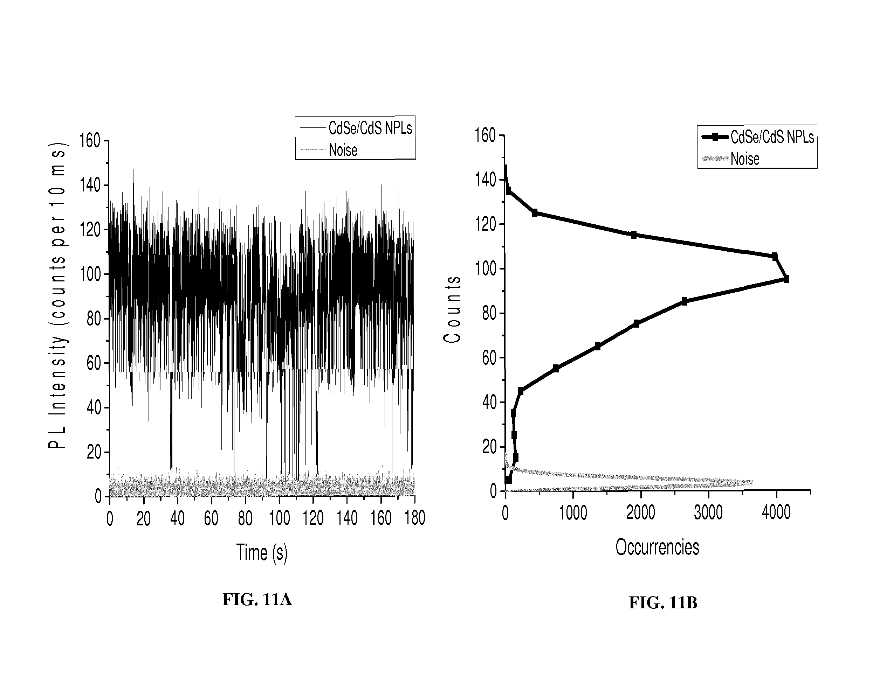

**Figures 11A** and **11B** show the emission time trace and corresponding normalized fluorescence intensity distribution of a single CdSe/CdS core/shell nanoplatelets with a shell thickness of 3 nm according to the present invention (trace in black and background noise in grey).

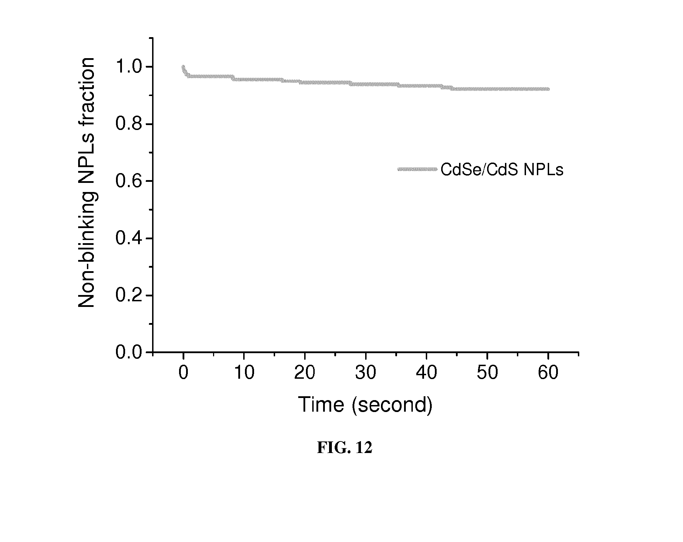

**Figure 12** shows the non-blinking fraction of CdSe/CdS core/shell nanoplatelets with a shell thickness of 5.5 nm according to the present invention (i.e. the fraction of continuously emissive nanoplatelets).

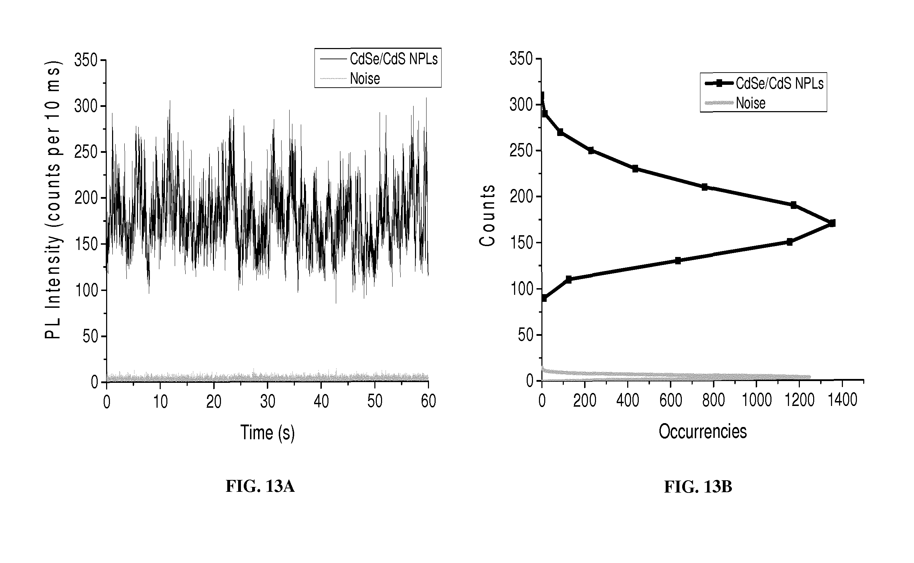

**Figures 13A** and **13B** show the emission time trace and corresponding normalized fluorescence intensity distribution of a single CdSe/CdS core/shell nanoplatelets with a shell thickness of 5.5 nm according to the present invention (trace in black and background noise in grey).

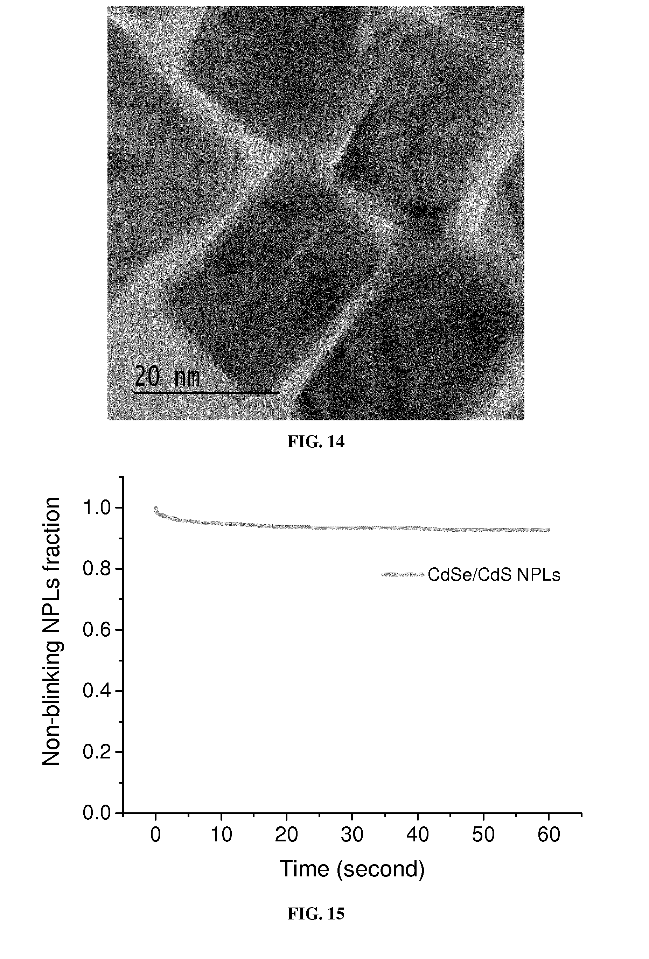

**Figure 14** shows TEM images of CdSe/CdS core/shell nanoplatelets with a shell thickness of 8.5 nm according to the present invention.
**Figure 15** shows the non-blinking fraction of CdSe/CdS core/shell nanoplatelets with a shell thickness of 8.5 nm according to the present invention (i.e. the fraction of continuously emissive nanoplatelets).

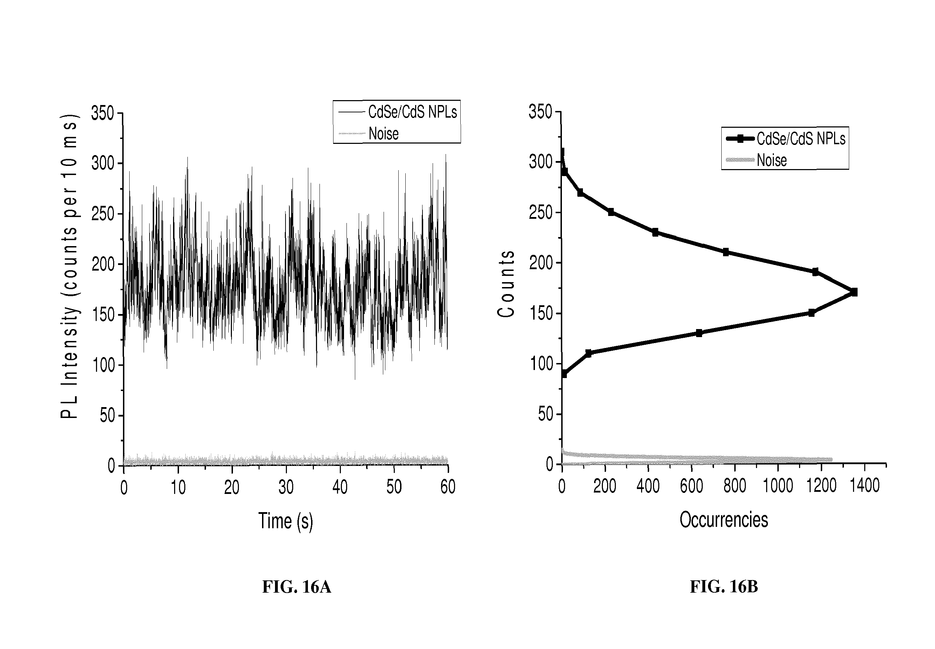

**Figures 16A** and **16B** show the emission time trace and corresponding normalized fluorescence intensity distribution of a single CdSe/CdS core/shell nanoplatelets with a shell thickness of 8.5 nm according to the present invention (trace in black and background noise in grey).

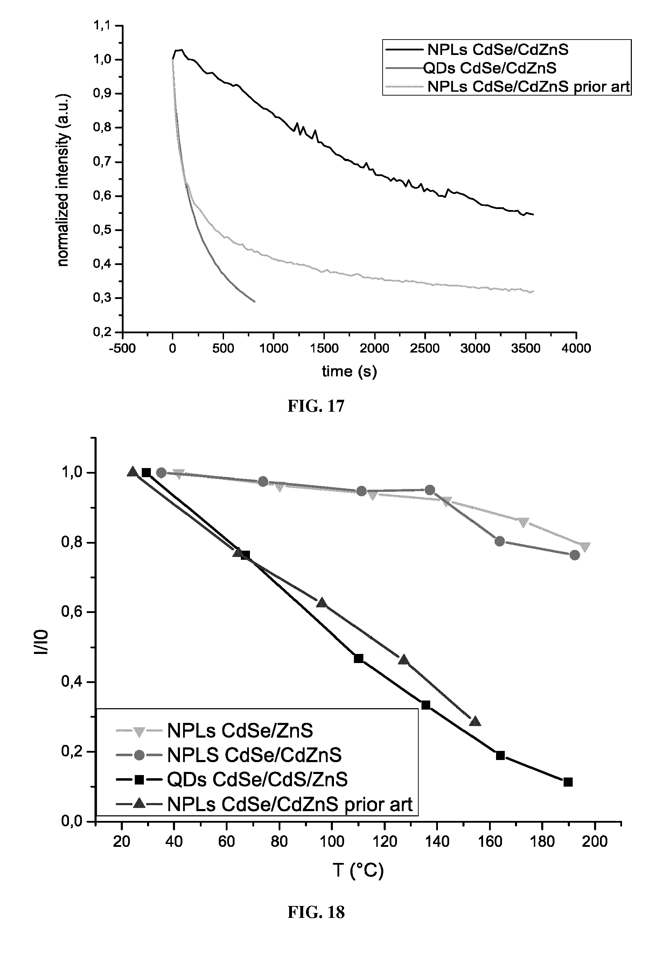

**Figure 17** shows the measurement of the normalized fluorescence quantum efficiency coming from film of CdSe/CdZnS nanoplatelets according to the invention, quantum dots of the prior art or CdSe/CdZnS nanoplatelets of the prior art deposed on microscope glass slides. Films are excited using a Hg lamp, and the emitted light is collected with an oil objective (100x, NA = 1.4) and adapted filters (550 nm short-pass filter for the excitation and 590 nm long-pass filter for the emission).
**Figure 18** shows the measurement of the normalized fluorescence quantum efficiency coming from CdSe/ZnS nanoplatelets according to the invention, CdSe/CdS/ZnS quantum dots according to the prior art and CdSe/CdZnS nanoplatelets according to the prior art deposed on a glass slide in function of temperature. Films are excited with a laser at 404 nm.

### EXAMPLES

### Nanoplatelets cores preparations

### Synthesis of CdSe 460 nanoplatelets (NPLs)

240 mg of Cadmium acetate (Cd(OAc)₂) (0,9mmol), 31 mg of Se 100 mesh, 150µL oleic acid (OA) and 15mL of 1-octadecene (ODE) are introduced in a three neck flask and are degassed under vacuum. The mixture is heated under argon flow at 180°C for 30 min.

### Synthesis of CdSe 510 NPLs

170mg of cadmium myristate (Cd(myr)₂) (0,3mmol), 12mg of Se 100 mesh and 15mL of ODE are introduced in a three neck flask and are degassed under vacuum. The mixture is heated under argon flow at 240°C, when the temperature reaches 195°C, 40mg of Cd(OAc)₂ (0,15mmol) are introduced. The mixture is heated for 10 minutes at 240°C.

### Synthesis of CdSe 550 NPLs

170mg of Cd(myr)₂ (0,3mmol) and 15mL of ODE are introduced in a three neck flask and are degassed under vacuum. The mixture is heated under argon flow at 250°C and 1mL of a dispersion of Se 100 mesh sonicated in ODE (0,1M) are quickly injected. After 30 seconds, 80mg of Cd(OAc)₂ (0,3mmol) are introduced. The mixture is heated for 10 minutes at 250°C.

### Synthesis of CdTe 428 NPLs

A three neck flask is charged with 130 mg of cadmium proprionate (Cd(prop)₂) (0.5 mmol), 80 µL of OA (0.25 mmol), and 10 mL of ODE, and the mixture is stirred and degassed under vacuum at 95°C for 2 h. The mixture under argon is heated at 180°C and 100 µL of a solution of 1 M Te dissolved in trioctylphosphine (TOP-Te) diluted in 0.5 mL of ODE are swiftly added. The reaction is heated for 20 min at the same temperature.

When 428 NPLs are prepared using Cd(OAc)₂, TOP-Te 1 M is injected between 120 and 140°C.

### Synthesis of CdTe 500 NPLs

A three-neck flask is charged with 130 mg of Cd(prop)₂ (0.5 mmol), 80 µL of OA (0.25 mmol), and 10 mL of ODE, and the mixture is stirred and degassed under vacuum at 95°C for 2 h. The mixture under argon is heated at 210°C, and 100 µL of a solution of 1 M TOP-Te diluted in 0.5 mL of ODE is swiftly added. The reaction is heated for 30 min at the same temperature.

When Cd(OAc)2 was used as cadmium precursor, TOP-Te is injected between 170 and 190°C.

### Synthesis of CdTe 556 NPLs

133 mg of Cd(OAc)₂ (0.5 mmol), 255 µL of OA (0.8 mmol), and 25 mL of ODE are charged into a three-neck flask, and the mixture is stirred and degassed under vacuum at 95°C for 2 h. The flask is filled with argon and the temperature is increased to 215°C. Then, 0.05 mmol of stoichiometric TOP-Te (2.24 M) diluted in 2.5 mL ODE is injected with a syringe pump at a constant rate over 15 min. When the addition is completed, the reaction is heated for 15 min.

### Synthesis of CdS 375 NPLs

In a three neck flask 160 mg of Cd(OAc)₂ (0.6mmol), 190 µL (0.6mmol) of OA, 1.5 mL of sulfur dissolved in 1-octadecene (S-ODE) 0.1M and 13.5 mL of ODE are introduced and degassed under vacuum for 30 minutes. Then the mixture is heated at 180°C under Argon flow for 30 minutes.

### Synthesis of CdS 407 NPLs

In a three neck flask 160 mg of Cd(OAc)₂ (0.6mmol), 190 µL (0.6mmol) of OA, 1.5 mL of S-ODE 0.1M and 13.5 mL of octadecene are introduced and degassed under vacuum for 30 minutes. Then the mixture is heated at 260°C under Argon flow for 1 minute.

### Synthesis of Core/Shell (Crown) CdSe/CdS NPLs

In a three neck flask, 320 mg of Cd(OAc)₂ (1.2 mmol), 380 µL of OA (1.51 mmol) and 8 mL of octadecene are degassed under vacuum at 65°C for 30 minutes. Then CdSe nanoplatelets cores in 4 mL of ODE are introduced under Argon. The reaction is heated at 210 °C and 0.3 mmol of S-ODE 0.05M are added drop wise. After injection, the reaction is heated at 210°C for 10 minutes.

### Synthesis of Core/Shell (Crown) CdSe/CdTe NPLs

In a three neck flask, CdSe nanoplatelets cores in 6 mL of ODE are introduced with 238 µL of OA (0.75 mmol) and 130 mg of Cd(prop)₂. The mixture is degassed under vacuum for 30 minutes then, under argon, the reaction is heated at 235°C and 50 µL of TOP-Te 1M in 1 mL of ODE is added drop wise. After the addition, the reaction is heated at 235°C for 15 minutes.

### Synthesis of CdSeS alloyed NPLs

170mg of Cd(myr)₂ (0,3mmol) and 15mL of ODE are introduced in a three neck flask and are degassed under vacuum. The mixture is heated under argon flow at 250°C and 1mL of a dispersion of Se 100 mesh sonicated in S-ODE and ODE (total concentration of selenium and sulfur 0,1M) are quickly injected. After 30 seconds, 120mg of Cd(OAc)₂ (0,45mmol) are introduced. The mixture is heated for 10 minutes at 250°C.

### Shells growth

### CdS shell growth

In a three neck flask, 15 mL of trioctylamine (TOA) are introduced and degassed under vacuum at 100°C. Then the reaction mixture is heated at 300°C under Argon and 5 mL of core nanoplatelets in ODE are swiftly injected followed by the injection of 7 mL of 0.1 M octanethiol solution in ODE and 7 mL of 0.1M Cd(OA)₂ in ODE with syringe pumps at a constant rate over 90 min. After the addition, the reaction is heated at 300°C for 90 minutes.

### ZnS shell growth

In a three neck flask, 15 mL of trioctylamine are introduced and degassed under vacuum at 100°C. Then the reaction mixture is heated at 300°C under Argon and 5 mL of core nanoplatelets in octadecene are swiftly injected followed by the injection of 7 mL of 0.1 M octanethiol solution in octadecene and 7 mL of 0.1M zinc oleate (Zn(OA)₂) in octadecene with syringe pumps at a constant rate over 90 min. After the addition, the reaction is heated at 300°C for 90 minutes.

### CdZnS gradient shell growth

In a three neck flask, 15 mL of trioctylamine are introduced and degassed under vaccum at 100°C. Then the reaction mixture is heated at 300°C under Argon and 5 mL of core nanoplatelets in octadecene are swiftly injected followed by the injection of 7 mL of 0.1 M octanethiol solution in octadecene with syringe pumps at a constant rate and 3.5 mL of 0.1M Cd(OA)₂ in octadecene and 3.5 mL of 0.1M Zn(OA)₂ in octadecene with syringe pumps at variables rates over 90 min. After the addition, the reaction is heated at 300°C for 90 minutes.

### CdZnS alloys shell growth

In a three neck flask, 15 mL of trioctylamine are introduced and degassed under vaccum at 100°C. Then the reaction mixture is heated at 300°C under Argon and 5 mL of core nanoplatelets in octadecene are swiftly injected followed by the injection of 7 mL of 0.1 M octanethiol solution in octadecene, 3.5 mL of 0.1M Cd(OA)₂ in octadecene and 3.5 mL of 0.1M Zn(OA)₂ in octadecene with syringe pumps at a constant rate over 90 min. After the addition, the reaction is heated at 300°C for 90 minutes.

### CdZnS shell growth(manufactured according to the prior art: ambient temperature

Mahler et al. JACS. 2012, 134(45), 18591-18598*)*

1 mL of CdSe 510 NPLs in hexane is diluted in 4 mL of chloroform, then 100 mg of thioacetamide (TAA) and 1 mL of octylamine are added in the flask and the mixture is sonicated until complete dissolution of the TAA (about 5 min). The color of the solution changed from yellow to orange during this time. 350 µL of a solution of Cd(NO3)2 0.2 M in ethanol and 150 µL of a solution of Zn(NO3)2 0.2 M in ethanol are then added to the flask. The reaction was allowed to proceed for 2 h at 65°C. After synthesis, the core/shell platelets were isolated from the secondary nucleation by precipitation with a few drops of ethanol and suspended in 5 mL of chloroform. Then 100 µL of Zn(NO3)2 0.2 M in ethanol is added to the nanoplatelets solution. They aggregate steadily and are resuspended by adding 200 µL oleic acid.

### ZnS alternative shell growth

In a three neck flask, 15 mL of trioctylamine are introduced and degassed under vacuum at 100°C. Then the reaction mixture is heated at 310°C under Argon and 5 mL of core nanoplatelets in octadecene mixed with 50µL of precursors mixture are swiftly injected followed by the injection of 2 mL of 0.1M zinc oleate (Zn(OA)₂) and octanethiol solution in octadecene with syringe pump at a constant rate over 80 min.

### Effect of ligand Exchange on Ouantum Yield

### Ligand exchange procedure (1-dodecanethiol)

Ligand exchange with 1-dodecanthiol have been done by treating 1 mL of core/shell nanoplatelets solution with 200 µL of 1-dodecanethiol. Then the solution is left without stirring at 65°C overnight. After, the exchanged nanoplatelets are washed by two successive precipitations by EtOH and resuspension in hexane (Table 1).

**Table 1**

| **Sample** | **Native NPLs** | **After ligand exchange¹** |
|---|---|---|
| NPLs CdSe/CdZnS | 75% | 69% |
| NPLs CdSe/CdS/ZnS | 72% | 65% |
| NPLs CdSe/ZnS | 74% | 72% |
| NPLs CdSe/CdZnS of the prior art | 58% | 10% |

| | | |
|---|---|---|
| ¹ Exchanged with 1-dodecanethiol. | | |

### Ligand exchange procedure (polymerized ligands)

1 mg of Core/shell nanoplatelets in hexane are precipitated with ethanol and centrifuged. The supernatant is removed and the nanoplatelets are dispersed in 200 µL of 3-mercaptopropionic acid (MPA). The mixture is sonicated to obtain a homogenous dispersion. The nanoplatelets dispersion is stored at 60°C for 2 hours. Then the nanoplatelets are centrifuged and the MPA phase is discarded. The nanoplatelets are dispersed in DMF under sonication and 2mg of potassium *tert*-butoxide are added and nanoplatelets dispersion is sonicated. The mixture is centrifuged and the DMF phase is discarded. The precipitated nanoplatelets are washed with ethanol and the nanoplatelets are dispersed in sodium tetraborate buffer. 200µL of aqueous solution of polymerized ligands, previously reduced 30 min with NaBH₄, are added to nanoplatelets dispersion. The solution is stored at 60°C overnight. The excess of free ligand and reagents are removed by Vivaspin. The evolution of the absorption spectrum and the emission spectrum of CdSe/CdZnS core/shell nanoplatelets after ligand exchange with a hydrosoluble polymer are for example shown in Figure 9. In addition, Table 2 exhibits the percentage (%) of Quantum yield on NPLs exchanged with polymerized ligands the following day and after 2 months.

**Table 2**

| **Sample** | **Native NPLs** | **After ligand exchange¹** | **2 months after ligand exchange²** |
|---|---|---|---|
| NPLs of the prior art | 20% | 7% | N.A. |
| NPLs CdSe/CdZnS | 75% | 69% | 62% |
| NPLs CdSe/ZnS | 68% | 65% | 60% |

| | | | |
|---|---|---|---|
| ¹ Exchanged with polymerized ligands. ² Stored at 4°C in the dark in a concentration of 10 µM. | | | |

### Layered material preparation:

A solution of CdSe-ZnS nanoplatelets is first precipitated in air free glove box by addition of ethanol. After centrifugation the formed pellet is redispersed in chloroform solution. Meanwhile a solution at 30 % in weight of Poly(maleic anhydride -alt-octadecene) (MW=40kg.mol⁻¹) in chloroform is prepared. Then the nanoplatelets solution is mixed with the polymer solution in a 1:1 volume ratio and the solution is further stirred. On an O₂ insulating substrate (glass or PET) the solution nanoplatelets-polymer mixture is brushed and let dried for 30min. Then UV polymerizable oligomer made of 99% of lauryl methacrylate and 1% of benzophenone is deposited on the top of the nanoplatelets film. A top substrate (same as the bottom substrate) is deposited on the system. The film is the polymerized under UV for 4 min. The layered material is then glued thanks to a PMMA solution dissolved in chloroform on a 455nm LED from Avigo technology. The LED is operated under a constant current ranging from 1mA to 500mA.

**Ensemble measurements:** The nanoplatelets in hexane solution are diluted in a mixture of 90% hexane/10% octane and deposited by drop-casting on a glass substrate. The sample is visualized using an inverted fluorescent microscope. An area of the sample containing several nanoplatelets is excited using a Hg lamp, and the emitted light is collected with an oil objective (100x, NA = 1.4) and adapted filters (550 nm short-pass filter for the excitation and 590 nm long-pass filter for the emission). The emitted light of the sample can be observed on a CCD camera (Cascade 512B, Roper Scientific) or directly through the microscope eyepiece with the naked eyes. A movie of the illuminated field containing at least 100 nanoplatelets is recorded for 1 minute at 33Hz frame rate. Using home-made software, the fluorescence intensity time traces of the emitting nanoplatelets are extracted as well as the noise of the background. By fixing an "off" threshold at 3 times the noise, the time of the first "off" event is computed for each time trace. Plotting the number of nanoplatelets which never went "off" over time give access to the global Non-blinking fraction of nanoplatelets over time.

**Single particle measurements:** The fluorescence intensity emission of unique nanoplatelets are recorded on a confocal microscope (Microtime 200, Picoquant) and a Hanbury Brown and Twist setup based on two avalanche photodiodes (SPAD PDM, MPD, time resolution 50 ps). The photodetection signal is recorded by a HydraHarp 400 module (Picoquant). In this configuration, the studied nanocrystal is excited with a pulsed diode emitting at 405 nm. To obtain a single nanoplatelet spectrum, a part or the totality of the collected photons is sent to an Andor shamrock 750 spectrometer. The dispersion system is a prism, and the detector is a CCD camera (Cascade 512B, Roper Scientific). Typical time traces of single nanoplatelets are obtained by integrating the number of photons collected over 10 ms.

### Photobleaching measurements in air

The nanoplatelets or quantum dots in hexane solution are diluted in a mixture of 90% hexane/10% octane and deposited by drop-casting on a glass substrate. The sample is visualized using an inverted fluorescent microscope. An area of the sample containing nanoplatelets or quantum dots as a concentration still allowing distinguishing single nanocrystals is excited using a Hg lamp, and the emitted light is collected with an oil objective (100x, NA = 1.4) and adapted filters (550 nm short-pass filter for the excitation and 590 nm long-pass filter for the emission). The emitted light of the sample can be observed on a CCD camera (Cascade 512B, Roper Scientific). An image of the illuminated field is taken every minute and the mean intensity of the film is normalized with the initial intensity, allowing to plot the mean intensity variations over time (see figure 17).

### Fluorescence stability versus temperature measurement

The layered material preparation is described above. The layered material is heated via a hot plate at the desired temperature ranging from 20°C to 200°C and the fluorescence is measured using an optical fiber spectrometer (Ocean-optics usb 2000) under excitation with a laser at 404nm. The measurements are taken after temperature stabilization (see figure 18).

## Claims

1. A population of fluorescent colloidal nanoplatelets, each member of the population comprising a nanoplatelet core including a first semiconductor material and a shell including a second semiconductor material on the surface of the nanoplatelet core, wherein at least 40% of the nanoplatelets of the population are continuously emissive for a period of at least one minute.

2. The population of fluorescent colloidal nanoplatelets according to claim **1,** wherein the shell of the nanoplatelet has a thickness of at least 3 nm.

3. The population of fluorescent colloidal nanoplatelets according to claim **1** or claim **2,** wherein after ligand exchange reaction the population exhibits a quantum yield decrease of less than 50%.

4. The population of semiconductor nanoplatelets according to anyone of claims **1** to **3,** wherein the population exhibits fluorescence quantum efficiency decrease of less than 50% after one hour under light illumination.

5. The population of semiconductor nanoplatelets according to anyone of claims **1** to **4,** wherein the population exhibits fluorescence quantum efficiency at 100 °C or above that is at least 80% of the fluorescence quantum efficiency of the population at 20°C.

6. The population of fluorescent colloidal nanoplatelets according to anyone of claims **1** to **5,** wherein the material composing the core and the shell comprises a material MxEy wherein:
M is selected from group Ib, IIa, IIb, IIIa, IIIb, IVa, IVb, Va, Vb, VIb, VIIb, VIII or mixtures thereof;
E is selected from group Va, VIa, VIIa or mixtures thereof; and
x and y are independently a decimal number from 0 to 5.

7. The population of fluorescent colloidal nanoplatelets according to anyone of claims **1** to **6,** wherein the material composing the core and the shell comprises a material MxEy, wherein:
M is Zn, Cd, Hg, Cu, Ag, Au, Ni, Pd, Pt, Co, Fe, Ru, Os, Mn, Tc, Re, Cr, Mo, W, V, Nd, Ta, Ti, Zr, Hf, Be, Mg, Ca, Sr, Ba, Al, Ga, In, Tl, Si, Ge, Sn, Pb, As, Sb, Bi, Sc, Y, La, Ce, Pr, Nd, Sm, Eu, Gd, Tb, Dy, Ho, Er, Tm, Yb, or a mixture thereof,
E is O, S, Se, Te, N, P, As, F, Cl, Br, I, or a mixture thereof, and
x and y are independently a decimal number from 0 to 5.

8. Use of a fluorescent colloidal nanoplatelet according to anyone of claims **1** to **7,** as a fluorophore.

9. A fluorophore conjugate comprising at least one fluorescent colloidal nanoplatelet according to anyone of claims **1** to **7,** associated to a specific-binding component.

10. The fluorophore conjugate according to claim **9,** wherein said specific-binding component is selected among antigens, steroids, vitamins, drugs, haptens, metabolites, toxins, environmental pollutants, amino acids, peptides, proteins, antibodies, polysaccharides, nucleotides, nucleosides, nucleic acids, nucleic acid polymers, carbohydrates, lipids, phospholipids, polymers, lipophilic polymers, polymeric microparticles, cells and virus.

11. Use of the fluorescent colloidal nanoplatelet according to anyone of claims **1** to **7** or the fluorophore conjugate according to claim **9** or claim **10** in a detection system.

12. A method for detecting an analyte in a sample, said method comprising:
- contacting said sample with a fluorophore conjugate according to claims **9** or **10,** wherein the specific-binding component is a binding partner for said analyte;
- incubating said conjugate with said sample for a sufficient amount of time for said analyte and said component to interact, thereby forming a fluorescent analyte; and
- illuminating said fluorescent analyte with an appropriate wavelength, whereby the presence of said analyte is determined in said sample.

13. A method for detecting multiple analytes in a sample, said method comprising:
- contacting said sample with multiples fluorophore conjugate according to claims **9** or **10,** wherein each specific-binding component is a binding partner for one analyte and wherein each fluorophore presents a different fluorescence emission;
- incubating said conjugates with said sample for a sufficient amount of time for said analytes and said components to interact, thereby forming multiple fluorescent analytes;
- illuminating said fluorescent analytes with an appropriate wavelength, whereby the presence of said analytes is determined in said sample.

14. A kit for detection of at least one analyte in a sample comprising at least one fluorescent colloidal nanoplatelet to anyone of claims **1** to **7** or at least one fluorophore conjugate according to anyone of claims **9** to **10.**

15. Use of the fluorescent colloidal nanoplatelet according to anyone of claims **1** to **7** or the fluorophore conjugate according to anyone of claims **9** to **10,** for in vivo animal imaging or for ex vivo live cells imaging, in fluorescence detection methods or as biosensors.
